(19) Europäisches Patentamt
European Patent Office
Office européen des brevets

(11) Numéro de publication: **0 502 043 B1**

# (12) FASCICULE DE BREVET EUROPEEN

(45) Date de publication de fascicule du brevet: **01.03.95**

(51) Int. Cl.6: **A61K 7/48**, A61K 7/06

(21) Numéro de dépôt: **90917311.4**

(22) Date de dépôt: **16.11.90**

(86) Numéro de dépôt internationale :
**PCT/FR90/00822**

(87) Numéro de publication internationale :
**WO 91/07945 (13.06.91 91/13)**

Le dossier contient des informations techniques présentées postérieurement au dépôt de la demande et ne figurant pas dans le présent fascicule.

## (54) UTILISATION DE XANTHINES, EVENTUELLEMENT INCORPOREES DANS DES LIPOSOMES, POUR FAVORISER LA PIGMENTATION DE LA PEAU OU DES CHEVEUX.

(30) Priorité: **28.11.89 FR 8915653**

(43) Date de publication de la demande:
**09.09.92 Bulletin 92/37**

(45) Mention de la délivrance du brevet:
**01.03.95 Bulletin 95/09**

(84) Etats contractants désignés:
**AT BE CH DE DK ES FR GB GR IT LI LU NL**

(56) Documents cités:
**FR-A- 2 609 395**
**US-A- 4 107 306**

**Cosmetics and toiletries, vol. 102, March 1987, Allured Publishing Corp. (New York, US), M. Duggan et al "Tyrosinase... The enzyme behind the tan", pages 97-101**

(73) Titulaire: **LVMH RECHERCHE**
**48-50, rue de Seine,**
**B.P. 79**
**F-92703 Colombes Cédex (FR)**

(72) Inventeur: **BONTE, Frédéric**
**5, place Charras**
**F-92400 Courbevoie (FR)**
Inventeur: **DUMAS, Marc**
**54, rue de l'Industrie**
**F-92700 Colombes (FR)**
Inventeur: **MEYBECK, Alain**
**Les Poissons-Apt 242**
**20 ter, rue de Bezons**
**F-92400 Courbevoie (FR)**
Inventeur: **MARECHAL, Christian**
**7, rue Charlot**
**F-75003 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

(74) Mandataire: **Portal, Gérard et al**
**Cabinet Beau de Loménie**
**158, rue de l'Université**
**F-75340 Paris Cédex 07 (FR)**

## Description

La présente invention concerne généralement l'utilisation de xanthines éventuellement incorporées, au moins en partie, dans des phases lamellaires lipidiques hydratées ou dans des liposomes pour la préparation de compositions cosmétiques ou pharmaceutiques favorisant la pigmentation de la peau ou des cheveux.

L'action de la théophylline, sur les mélanocytes en culture, a déjà été étudiée (Leising H.B. et coll., Z. Naturforsch. 1977 32C (7-8) 567-72). Cette étude, limitée à une expérimentation in vitro, a révélé que l'activité de stimulation de la mélanogénèse par la théophylline est faible, de sorte qu'elle a dissuadé l'utilisation commerciale de ce composé dans cette application.

Pour obtenir des compositions pigmentantes présentant une activité satisfaisante, on a cherché à réaliser des synergies par l'association de diverses substances. Ainsi la demande de brevet JP-62 045 527 décrit des compositions pour la prévention et le traitement des cheveux gris à base d'AMP cyclique associé à diverses substances telles que papaverine, théophylline ou isobutylméthylxanthine.

Par ailleurs, l'encapsulation en liposomes de la théophylline a déjà été décrite. En particulier, la demande de brevet Inchema SA DE-A-2 249 552, également citée dans Chemical Abstracts volume 79, n° 8 référence 45830t, décrit l'encapsulation dans des liposomes de substances biologiquement actives, notamment la théophylline. Toutefois dans ce document, il n'est nullement fait mention de l'activité des substances concernées sur la pigmentation de la peau.

Il a maintenant été découvert, de manière totalement inattendue, et ceci constitue la base de la présente invention, que certaines xanthines ou des extraits végétaux en contenant présentaient une activité intéressante sur la pigmentation de la peau ou des cheveux. Il a en outre été découvert que cette activité peut être fortement potentialisée par leur incorporation dans des phases lamellaires lipidiques hydratées ou dans des liposomes.

Il a également été observé, de manière inattendue, que l'application sur la peau d'une composition contenant l'une de ces xanthines, provoquait un épaississement de l'épiderme après exposition au rayonnement solaire de façon plus importante qu'en l'absence de toute application. Ainsi, cet effet, s'ajoutant à celui obtenu, indépendamment, sur la pigmentation cutanée permet de renforcer les défenses naturelles de la peau contre les radiations solaires.

Ainsi, selon un premier aspect, la présente invention concerne l'utilisation d'une xanthine de formule I suivante :

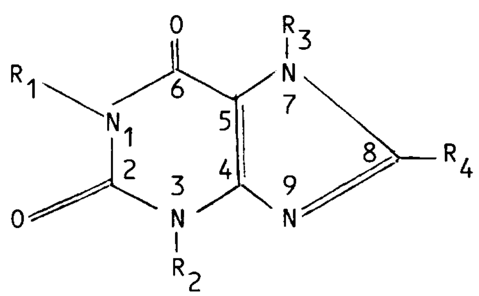

(I)

dans laquelle :

$R_1$ et $R_2$ sont identiques ou différents et représentent chacun un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle;

$R_3$ représente un atome d'hydrogène, un radical alkyle ayant de préférence de 1 à 4 atomes de carbone, un radical benzyle, ledit radical alkyle étant éventuellement substitué par un reste hétérocyclique tel que le reste 4-morpholinyle ou 1-pipéridinyle, ou par un ou plusieurs atomes d'halogène ou groupes hydroxy;

$R_4$ représente un atome d'hydrogène ou d'halogène, un radical alkyle à chaîne droite, ramifiée ou cyclique, ayant de préférence de 1 à 6 atomes de carbone, un radical aryle ou un groupe

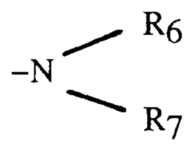

dans lequel $R_6$ et $R_7$ représentent indépendamment un atome d'hydrogène, un radical alkyle ayant de 1 à 6 atomes de carbone, de préférence un radical méthyle ou éthyle, ou bien forment ensemble et avec l'atome d'azote un hétérocycle de préférence monocyclique saturé, lesdits radicaux alkyle ou aryle étant éventuellement substitués par un ou plusieurs atomes d'halogène ou groupes hydroxy, méthoxy, carbométhoxy ou amino;

ou l'utilisation d'un sel pharmaceutiquement acceptable de cette xanthine, ou celle d'un extrait végétal contenant une telle xanthine;

pour la préparation d'une composition pharmaceutique, notamment dermatologique, destinée à favoriser la pigmentation de la peau ou des cheveux ou à renforcer les défenses naturelles de la peau contre les rayonnements solaires.

Selon un deuxième aspect, la présente invention concerne aussi l'utilisation d'une xanthine de Formule (I) suivante :

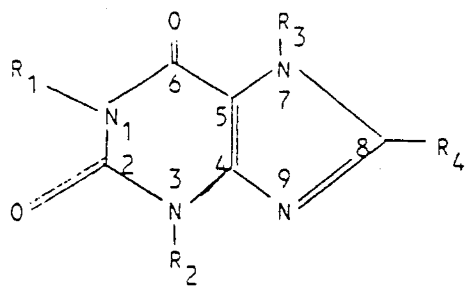

(I)

dans laquelle :

$R_1$ et $R_2$ sont identiques ou différents et représentent chacun un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle;

$R_3$ représente un atome d'hydrogène, un radical alkyle ayant de préférence de 1 à 4 atomes de carbone, un radical benzyle, ledit radical alkyle étant éventuellement substitué par un reste hétérocyclique tel que le reste 4-morpholinyle ou 1-pipéridinyle, ou par un ou plusieurs atomes d'halogène ou groupes hydroxy;

$R_4$ représente un atome d'hydrogène ou d'halogène, un radical alkyle à chaîne droite, ramifiée ou cyclique, ayant de préférence de 1 à 6 atomes de carbone, un radical aryle ou un groupe

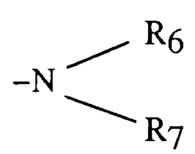

dans lequel $R_6$ et $R_7$ représentent indépendamment un atome d'hydrogène, un radical alkyle ayant de 1 à 6 atomes de carbone, de préférence un radical méthyle ou éthyle, ou bien forment ensemble et avec l'atome d'azote un hétérocycle de préférence monocyclique saturé, lesdits radicaux alkyle ou aryle étant éventuellement substitués par un ou plusieurs atomes d'halogène ou groupes hydroxy, méthoxy, carbométhoxy ou amino;

ou l'utilisation d'un sel cosmétiquement acceptable de cette xanthine, ou celle d'un extrait végétal contenant une telle xanthine;

comme agent cosmétique de pigmentation de la peau ou des cheveux ou de renforcement des

défenses naturelles de la peau contre les rayonnements solaires.

Selon un mode de réalisation particulier de l'invention, la xanthine utilisée pour ladite préparation d'une composition cosmétique ou pharmaceutique, notamment dermatologique, est une xanthine de Formule (I) précitée dans laquelle :

$R_1$ représente un atome d'hydrogène ou un radical méthyle, éthyle, propyle, butyle, isobutyle, pentyle, isopentyle, 2-propényle, 2-propynyle, benzyle, hydroxyméthyle, 2-hydroxyéthyle, 2-hydroxypropyle ou 3-hydroxypropyle, $R_2$ représente un atome d'hydrogène ou un radical méthyle, éthyle, propyle, isopropyle, butyle, isobutyle, 2-propényle, 2-méthyl-2-propényle,cyclohexyle, benzyle ou 2,3-dibromopropyle,

$R_3$ représente un atome d'hydrogène ou un radical méthyle, chlorométhyle, hydroxyméthyle, 2-chloroéthyle, 4-morpholinylméthyle ou benzyle,

$R_4$ représente un atome d'hydrogène ou de brome, ou un radical méthyle, éthyle, cyclopropyle, cyclopentyle, cyclohexyle, tertiobutyle, 2-carbométhoxyéthyle, hydroxyméthyle, méthoxyméthyle, méthylamino, 1-pipéridinyle.

Parmi les xanthines de formule I précitée, utilisées selon ledit mode de réalisation particulier, on peut citer par exemple les xanthines suivantes :

Xanthine,
1,8-diméthyl-3-isobutyl-xanthine,
7-benzyl-1,8-diméthyl-3-isobutyl-xanthine,
1(2-hydroxyéthyl)-3-isobutyl-8-méthyl-xanthine,
1,8-diméthyl-3-(2-méthyl-2-propényl)-xanthine,
3-(2,3-dibromopropyl)-1-éthyl-8-méthyl-xanthine,
1,3-di(2-propényl)-8-méthyl-xanthine,
7-benzyl-8-bromo-3-isobutyl-1-méthyl-xanthine,
8-éthyl-1-(2-hydroxyéthyl)-3-isobutyl-xanthine,
8-hydroxyméthyl-3-isobutyl-1-méthyl-xanthine,
3-isobutyl-8-méthoxyméthyl-1-méthyl-xanthine,
8-(2-carbométhoxyéthyl)-3-isobutyl-1-méthyl-xanthine,
3-isobutyl-1-méthyl-8-tertio-butyl-xanthine,
3-isobutyl-1-méthyl-8-méthylamino-xanthine,
1,3-dipropyl-8-(1-pipéridinyl)-xanthine,
8-cyclopropyl-1,3-dipropyl-xanthine,
8-cyclopentyl-1,3-dipropyl-xanthine,
8-cyclohexyl-1,3-dipropyl-xanthine,
1,3-diméthyl-8-phényl-xanthine,
1,3-dipropyl-8-phényl-xanthine,
8-(2-amino-4-chlorophényl)-1,3-dipropyl-xanthine,
1,3-diéthyl-7-méthyl-xanthine,
1,3-dipropyl-7-méthyl-xanthine,
7-chlorométhyl-1,3-diméthyl-xanthine,
1,3-diméthyl-7-hydroxyméthyl-xanthine,
1,3-diméthyl-7-(4-morpholinylméthyl)-xanthine,
1,3,7-triéthyl-xanthine,
3,7-diéthyl-1-(2-propynyl)-xanthine,
7-(2-chloroéthyl)-1,3-diméthyl-xanthine.

Les xanthines préférées de la précédente liste pour la mise en oeuvre de la présente invention sont :

1,8-diméthyl-3-isobutyl-xanthine,
8-(2-carbométhoxyéthyl)-3-isobutyl-xanthine, et
3-isobutyl-1-méthyl-8-tertiobutyl-xanthine.

Cependant, il a maintenant été découvert de façon tout à fait inattendue qu'une xanthine de formule I précitée dans laquelle :

$R_3$ et $R_4$ représentent chacun un atome d'hydrogène, confère à la composition cosmétique ou pharmaceutique, notamment dermatologique, dans laquelle elle est incorporée, une activité plus importante sur la pigmentation de la peau ou des cheveux ou sur le renforcement des défenses naturelles de la peau contre les rayonnements solaires.

Ainsi, selon un mode de réalisation préféré de l'invention, la xanthine utilisée pour la préparation d'une composition cosmétique ou pharmaceutique, notamment dermatologique, est une xanthine de formule I précitée dans laquelle : $R_1$ et $R_2$ représentent chacun, indépendamment l'un de l'autre, un atome d'hydrogène ou un radical tel que défini plus haut, et $R_3$ et $R_4$ représentent chacun un atome d'hydrogène.

A titre d'exemple, concernant ce mode de réalisation préféré, on peut citer les xanthines suivantes :

3-isobutyl-1-méthyl-xanthine,
3-benzyl-1-méthyl-xanthine,
3-isopentyl-1-méthyl-xanthine,
1-méthyl-3-(2-propényl)-xanthine,
1-méthyl-3-(2-méthyl-2-propényl)-xanthine,
3-butyl-1-méthyl-xanthine,
1,3-diéthyl-xanthine,
1-éthyl-3 propyl-xanthine,
3-butyl-1-éthyl-xanthine,
1-éthyl-3-(2-méthyl-2-propényl)-xanthine,
3-cyclohexyl-1-éthyl-xanthine,
1-(2-hydroxyéthyl)-3-isobutyl-xanthine,
1,3-dipropyl-xanthine,
1,3-di(2-propényl)-xanthine,
1,3-dibutyl-xanthine,
3-éthyl-1-pentyl-xanthine,
1-benzyl-3-isobutyl-xanthine.

Parmi les xanthines de la liste ci-dessus, celles dont l'utilisation selon la présente invention est préférée sont :

3-isobutyl-1-méthyl-xanthine,
3-benzyl-1-méthyl-xanthine,
1,3-diéthyl-xanthine;
1-éthyl-3-propyl-xanthine,
3-butyl-1-éthyl-xanthine,
1,3-dipropyl-xanthine,
1,3-dibutyl-xanthine.

Selon une variante de réalisation, la xanthine précitée est choisie parmi la 1,3-diméthylxanthine ou la 3,7-diméthylxanthine.

Selon une autre variante de réalisation, la xanthine précitée est la 3-isobutyl-1-méthylxanthine.

L'invention concerne encore l'utilisation d'une xanthine ou l'un de ses sels pharmaceutiquement acceptables de Formule (Ia):

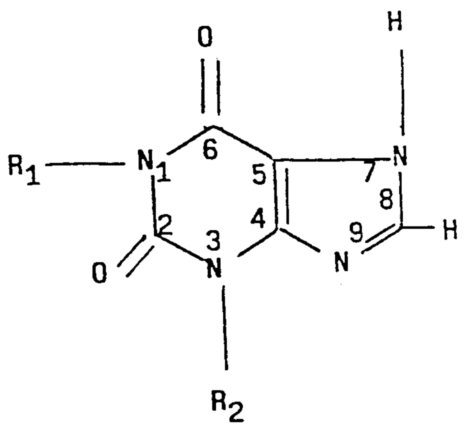

(Ia)

dans laquelle :

$R_1$ et $R_2$ sont différents et représentent chacun indépendamment un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle; l'un parmi $R_1$ ou $R_2$ étant un atome d'hydrogène;

ou au moins un extrait végétal contenant une xanthine de Formule (Ia), ou une combinaison des composés et extraits précités;

pour la préparation d'une composition pharmaceutique, notamment dermatologique, destinée à favoriser la pigmentation de la peau ou des cheveux ou à renforcer les défenses naturelles de la peau contre les rayonnements solaires.

Selon encore un quatrième aspect, l'invention concerne l'utilisation d'une xanthine ou l'un de ses sels cosmétiquement acceptables, de Formule (Ia):

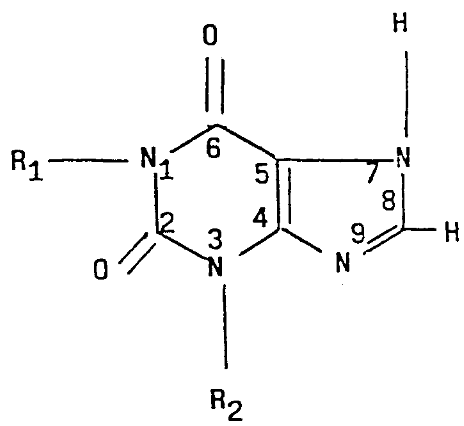

(Ia)

dans laquelle:

$R_1$ et $R_2$ sont différents et représentent chacun independamment un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle ; l'un parmi $R_1$ ou $R_2$ étant un atome d'hydrogène, ou au moins un extrait végétal contenant une xanthine de Formule (Ia), ou la combinaison des composés et extraits ci-dessus, dans un excipient, véhicule ou support cosmétiquement acceptable,

comme agent cosmétique de pigmentation de la peau ou des cheveux ou de renforcement des défenses naturelles de la peau contre les rayonnements solaires.

En particulier, $R_2$ peut représenter un atome d'hydrogène et $R_1$ représente un radical alkyle ayant de préférence de 1 à 4 atomes de carbone, notamment un radical méthyle, éthyle, propyle ou butyle, de préférence un radical méthyle.

Enfin, selon un mode de réalisation de la présente invention encore préféré, $R_1$ représente un atome d'hydrogène et $R_2$ représente un radical alkyle ayant de préférence de 1 à 4 atomes de carbone. De préférence, dans ce cas, $R_2$ représente un radical méthyle, propyle, butyle ou isobutyle.

Selon la présente invention, les xanthines précitées peuvent être obtenues par synthèse. Certaines sont disponibles dans le commerce. Comme plantes à partir desquelles on peut obtenir les xanthines, on citera en particulier la graine de cacaoyer, les feuilles de thé, le maté (= Ilex paraquariensis), les graines de cola, en particulier Cola nitida et Cola verticillata. Des procédés de synthèse de xanthines sont décrits dans DE 1 245 969 ; Ann. Chem. (1966), Vol. 697, pp. 142 à 158; Chem. Ber. (1953), Vol. 86, p. 321-33 ; J. Chem. Soc. (1950), P. 1 884-8 ; J. Org. Chem. (1980), p. 1 711-13, Vol. 45, No. 9; GB 982 079; DD 8 957 ; SU 115 947 (Chem. Abstr. Vol. 53, 15 107-H), J. Gen. Chem. (USSR), (1946), Vol. 16, p. 179-86 (Chem. Abstr. Vol. 41, 96-d) ; J. Applied Chemistry (1940), Vol. 13, p 1641-3 ; Chem. Ber. (1947), Vol 80, p. 401-5 ; WO-A2-87/04435 ; Bull. Chem. Soc. Japan (1973), Vol 46, No. 2, pp. 506-509 ; J. Chem. Soc. (1962), p. 1866.

Les sels pharmaceutiquement ou cosmétiquement acceptables des xanthines de Formule (I) sont des sels non toxiques pour l'homme dans le cadre des utilisations selon l'invention, comme par exemple des sels alcalins, tels que sels de sodium, de potassium ou d'ammonium, des sels de bases organiques telles que l'éthanolamine, la diéthanolamine, l'éthylènediamine, l'isopropylamine, la triéthanolamine, l'octadécylamine ou la choline, ou un sel d'acide aminé tel que la glycine ou la lysine. Ces sels sont obtenus par des procédés classiques, bien connus de l'homme de l'art, par exemple en faisant réagir la xanthine avec la

base, généralement en présence de solvant.

Suivant un mode particulier de réalisation de la présente invention, on utilise au moins une xanthine de Formule (I) ou (Ia) précitée ou l'un de ses sels tels que définis précédemment, en combinaison avec au moins un extrait de plante précité.

Selon une caractéristique particulière de l'invention, la concentration totale en composés de Formule (I) ou (Ia) précitée, ou de leurs sels, ou des extraits végétaux les contenant est comprise entre 0,001 % et 10 % en poids, de préférence entre 0,01 et 1 % en poids, par rapport au poids total de la composition.

Selon un autre mode de réalisation de l'invention, le composé de Formule (I) ou (Ia) précitée ou l'un de ses sels cosmétiquement ou pharmaceutiquement acceptables ou l'extrait végétal contenant un tel composé, ou une combinaison des composé et extraits précités, est au moins en partie incorporé dans des phases lamellaires lipidiques hydratées ou dans des vésicules de type liposomes.

Selon un cinquième aspect, la présente invention concerne également une composition cosmétique ou pharmaceutique, notamment dermatologique, notamment destinée à favoriser la pigmentation de la peau ou des cheveux, caractérisée en ce qu'elle comprend au moins une xanthine de Formule (Ia) ou de l'un de ses sels cosmétiquement ou pharmaceutiquement acceptables, de formule :

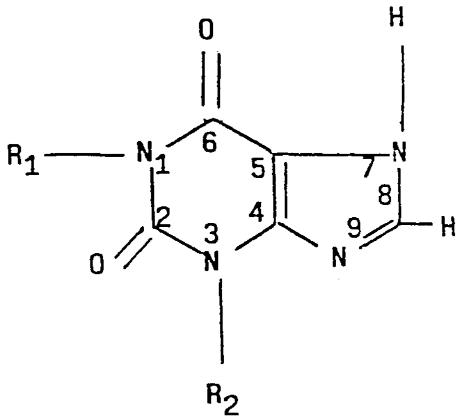

(Ia)

dans laquelle :

$R_1$ et $R_2$ sont différents et représentent chacun indépendamment un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle ; l'un parmi $R_1$ ou $R_2$ étant un atome d'hydrogène ;

ou au moins un extrait végétal contenant une xanthine de Formule (Ia), ou une combinaison des composés et extraits ci-dessus,

dans un excipient, véhicule ou support cosmétiquement ou pharmaceutiquement acceptable.

Selon encore un sixième aspect, la présente invention concerne encore une composition cosmétique, ou pharmaceutique, notamment dermatologique, notamment destinée à favoriser la pigmentation de la peau ou des cheveux, caractérisée en ce qu'elle comprend une quantité efficace pour la production de la pigmentation d'au moins une xanthine choisie parmi le groupe consistant de :

1,8-diméthyl-3-isobutylxanthine,
7-benzyl-1,8-diméthyl-3-isobutylxanthine,
1-(2-hydroxyéthyl)-3-isobutyl-8-méthylxanthine
1,8-diméthyl-3-(2-méthyl-2-propényl)xanthine,
3-(2,3-dibromopropyl)-1-éthyl-8-méthylxanthine,
1,3-di(2-propényl)-8-méthylxanthine,
7-benzyl-8-bromo-3-isobutyl-1-méthylxanthine,
8-éthyl-1-(2-hydroxyéthyl)-3-isobutylxanthine,
8-hydroxyméthyl-3-isobutyl-1-méthylxanthine,
3-isobutyl-8-méthoxyméthyl-1-méthylxanthine,

8-(2-carbométhoxyéthyl)-3-isobutyl-1-méthylxanthine,
3-isobutyl-1-méthyl-8-tert-butylxanthine,
3-isobutyl-1-méthyl-8-méthylaminoxanthine,
1,3-dipropyl-8(pipéridin-1-yl)xanthine,
8-cyclopropyl-1,3-dipropylxanthine,
8-cyclopentyl-1,3-dipropylxanthine,
8-cyclohexyl-1,3-dipropylxanthine,
1,3-diméthyl-8-phénylxanthine,
1,3-dipropyl-8-phénylxanthine,
8-(2-amino-4-chlorophényl)-1,3-dipropylxanthine,
1,3-diéthyl-7-méthylxanthine,
1,3-dipropyl-7-méthylxanthine,
7-chlorométhyl-1,3-diméthylxanthine,
1,3-diméthyl-7-hydroxyméthylxanthine,
1,3-diméthyl-7-(morpholin-4-yl-méthyl)xanthine,
1,3,7-triéthylxanthine,
3,7-diéthyl-1-(2-propényl)xanthine,
7-(2-chloroéthyl)-1,3-diméthylxanthine,
3-benzyl-1-méthylxanthine,
3-isopentyl-1-méthylxanthine,
1-méthyl-3-(2-propényl)xanthine,
1-méthyl-3-(2-méthyl-2-propényl)xanthine,
3-butyl-1-méthylxanthine,
1,3-diéthylxanthine,
1-éthyl-3-propylxanthine,
3-butyl-1-éthylxanthine,
1-éthyl-3-(2-méthyl-2-propényl)xanthine,
3-cyclohéxyl-1-éthylxanthine,
1-(2-hydroxyéthyl)-3-isobutylxanthine,
1,3-dipropylxanthine,
1,3-di-(2-propénylxanthine,
1,3-dibutylxanthine,
3-éthyl-1-penthylxanthine,
1-benzyl-3-isobutylxanthine,
1-méthylxanthine,
1-éthylxanthine,
1-propylxanthine,
1-butylxanthine,
3-méthylxanthine,
3-propylxanthine,
3-butylxanthine, et
3-isobutylxanthine,

ou un sel pharmaceutiquement ou cosmétiquement acceptable de cette xanthine, au moins un extrait végétal contenant une telle xanthine, ou une combinaison des composés et extraits ci-dessus,

dans un excipient, véhicule ou support cosmétiquement ou pharmaceutiquement acceptable.

Selon un mode de réalisation avantageux, le composé de Formule (Ia) ou l'un de ses sels tels que précédemment définis, ou au moins un extrait végétal contenant un composé de Formule (Ia) précitée, ou une combinaison des composé et extraits précités, est incorporé au moins en partie dans des phases lamellaires lipidiques hydratées ou dans des vésicules de type liposomes, dans un excipient, véhicule ou support cosmétiquement ou pharmaceutiquement acceptable.

D'une façon générale, la concentration totale des composés, de leurs sels, ou des extraits utilisés conformément à la présente invention, non incorporés ou incorporés au moins en partie dans des phases lamellaires lipidiques hydratées ou dans des vésicules type liposome, est comprise entre 0,001 % et 10 % en poids, de préférence entre 0,01 % et 1 % en poids, par rapport au poids total de la composition finale. Les compositions cosmétiques ou pharmaceutiques, notamment dermatologiques, selon l'invention, peuvent être appliquées par voie topique pour favoriser la pigmentation de la peau et des cheveux ou pour renforcer les défenses naturelles de la peau, en particulier comme des compositions se présentant sous forme de crème, de gel ou de lotion destiné à l'application topique sur la peau ou les cheveux.

Ainsi, les compositions cosmétiques ou pharmaceutiques, notamment dermatologiques, selon l'invention, trouvent diverses applications en cosmétologie ou en dermatologie, dès lors que l'augmentation de pigmentation ou le renforcement des défenses naturelles de la peau ou des cheveux sont recherchés. Par exemple, ces compositions peuvent être utilisées comme produit solaire pour accélérer ou intensifier le bronzage ce qui, outre l'avantage esthétique souvent recherché, permet de renforcer les défenses naturelles contre le rayonnement ultraviolet par l'augmentation du taux de mélanine dans l'épiderme et son épaississement. Ces compositions peuvent encore être utilisées, par exemple sous forme de crème, pour donner à la peau un aspect plus hâlé, ou encore, sous forme de lotion, pour la prévention et le traitement des cheveux gris. Par ailleurs, en dermatologie, les compositions selon la présente invention peuvent être utilisées comme agent thérapeutique, seules ou en association avec d'autres médicaments, en particulier en administration topique dans le traitement des disfonctionnements de la mélanogénèse, par exemple pour soigner le vitiligo.

Egalement, selon un septième aspect, la présente invention concerne encore un procédé de fabrication d'une composition cosmétique ou pharmaceutique, notamment dermatologique, destiné à favoriser la pigmentation de la peau ou des cheveux, caractérisé en ce qu'il comprend l'incorporation au moins partielle d'au moins un composé de Formule (I) ou de l'un de ses sels tels que précédemment définis, ou d'au moins un extrait végétal le contenant, ou d'une combinaison des composés et extraits précités, dans un excipient, véhicule ou support cosmétiquement ou pharmaceutiquement acceptable.

Selon un huitième aspect, la présente invention couvre encore un procédé de fabrication d'une composition cosmétique ou pharmaceutique, notamment dermatologique, destinée à favoriser la pigmentation de la peau ou des cheveux, caractérisé en ce qu'il comprend au préalable l'incorporation au moins partielle d'au moins un composé de Formule (I) ou de l'un de ses sels tels que précédemment définis, ou d'au moins un extrait végétal le contenant, ou d'une combinaison des composés et extraits précités, dans des phases lamellaires lipidiques hydratées ou dans des vésicules de type liposome, que l'on mélange ensuite avec un excipient, véhicule ou support cosmétiquement ou pharmaceutiquement acceptable.

Selon un neuvième aspect, la présente invention vise à couvrir un procédé de traitement cosmétique de la peau ou de la chevelure pour promouvoir la pigmentation, ledit procédé comprenant l'application à la peau ou à la chevelure à pigmenter d'une quantité efficace pour produire la pigmentation, d'au moins une xanthine de Formule (I) suivante :

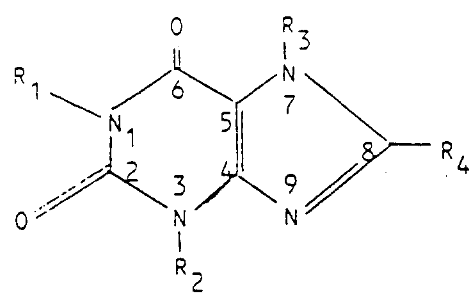

(I)

dans laquelle :

$R_1$ et $R_2$ sont identiques ou différents et représentent chacun un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle ;

$R_3$ représente un atome d'hydrogène, un radical alkyle ayant de préférence de 1 à 4 atomes de carbone, un radical benzyle, ledit radical alkyle étant éventuellement substitué par un reste hétérocyclique tel que le reste 4-morpholinyle ou 1-pipéridinyle, ou par un ou plusieurs atomes d'halgène ou groupes hydroxy ;

$R_4$ représente un atome d'hydrogène ou d'halogène, un radical alkyle à chaîne droite, ramifiée ou cyclique, ayant de préférence de 1 à 6 atomes de carbone, un radical aryle ou un groupe

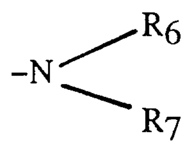

dans lequel $R_6$ et $R_7$ représentent indépendamment un atome d'hydrogène, un radical alkyle ayant de 1 à 6 atomes de carbone, de préférence un radical méthyle ou éthyle, ou bien forment ensemble et avec l'atome d'azote un hétérocycle de préférence monocyclique saturé, lesdits radicaux alkyle ou aryle étant éventuellement substitués par un ou plusieurs atomes d'halogène ou groupes hydroxy, méthoxy, carbométhoxy ou amino ;

ou d'un sel cosmétiquement acceptable de cette xanthine, ou d'un extrait végétal contenant une telle xanthine.

Selon un dixième aspect, la présente invention vise également à couvrir un procédé de traitement cosmétique de la peau ou de la chevelure pour promouvoir la pigmentation, ledit procédé comprenant l'application à la peau ou à la chevelure à pigmenter d'une quantité efficace pour produire la pigmentation, d'au moins une xanthine de Formule (Ia) suivante :

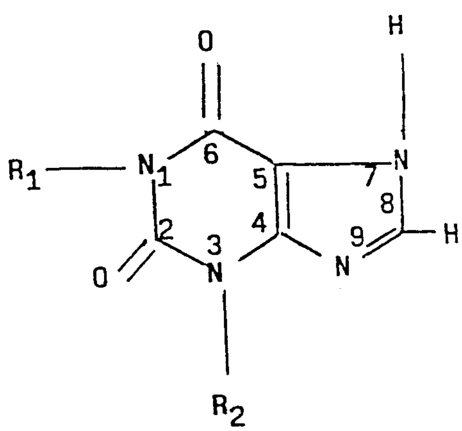

(Ia)

:

dans laquelle

$R_1$ et $R_2$ sont différents et représentent chacun indépendamment un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle ;

l'un parmi $R_1$ et $R_2$ étant un atome d'hydrogène,

ou l'un de ses sels cosmétiquement acceptables.

Selon encore un onzième aspect, la présente invention vise à couvrir un procédé de traitement cosmétique de la peau ou de la chevelure pour promouvoir la pigmentation, ledit procédé comprenant l'application à la peau ou à la chevelure à pigmenter d'une quantité efficace pour produire la pigmentation, d'au moins une xanthine, choisie parmi le groupe consistant de :

1,8-diméthyl-3-isobutylxanthine,
7-benzyl-1,8-diméthyl-3-isobutylxanthine,
1-(2-hydroxyéthyl)-3-isobutyl-8-méthylxanthine,
1,8-diméthyl-3-(2-méthyl-2-propényl)xanthine,
3-(2,3-dibromopropyl)-1-éthyl-8-méthylxanthine,
1,3-di(2-propényl)-8-méthylxanthine,
7-benzyl-8-bromo-3-isobutyl-1-méthylxanthine,
8-éthyl-1-(2-hydroxyéthyl)-3-isobutylxanthine,
8-hydroxyméthyl-3-isobutyl-1-méthylxanthine,

3-isobutyl-8-méthoxyméthyl-1-méthylxanthine,
8-(2-carbométhoxyéthyl)-3-isobutyl-1-méthylxanthine,
3-isobutyl-1-méthyl-8-tert-butylxanthine,
3-isobutyl-1-méthyl-8-méthylaminoxanthine,
1,3-dipropyl-8(pipéridin-1-yl)xanthine,
8-cyclopropyl-1,3-dipropylxanthine,
8-cyclopentyl-1,3-dipropylxanthine,
8-cyclohexyl-1,3-dipropylxanthine,
1,3-diméthyl-8-phénylxanthine,
1,3-dipropyl-8-phénylxanthine,
8-(2-amino-4-chlorophényl)-1,3-dipropylxanthine,
1,3-diéthyl-7-méthylxanthine,
1,3-dipropyl-7-méthylxanthine,
7-chlorométhyl-1,3-diméthylxanthine,
1,3-7-hydroxyméthylxanthine,
1,3-diméthyl-7-morpholin-4-yl-méthyl)xanthine,
1,3,7-triéthylxanthine,
3,7-diéthyl-1-(2-propényl) xanthine,
7-(2-chloroéthyl)-1,3-diméthylxanthine,
3-benzyl-1-méthylxanthine,
3-isopentyl-1-méthylxanthine,
1-méthyl-3-(2-propényl)xanthine,
1-méthyl-3-(2-méthyl-2-propényl)xanthine,
3-butyl-1-méthylxanthine,
1,3-diéthylxanthine,
1-éthyl-3-propylxanthine,
3-butyl-1-éthylxanthine,
1-éthyl-3-(2-méthyl-2-propényl)xanthine,
3-cyclohexyl-1-éthylxanthine,
1-(2-hydroxyéthyl)-3-isobutylxanthine,
1,3-dipropylxanthine,
1,3-di-(2-propényl)xanthine,
1,3-dibutylxanthine,
3-éthyl-1-pentylxanthine,
1-benzyl-3-isobutylxanthine,
1-méthylxanthine,
1-éthylxanthine,
1-propylxanthine,
1-butylxanthine,
3-méthylxanthine,
3-propylxanthine,
3-butylxanthine, et
3-isobutylxanthine,

ou un sel cosmétiquement acceptable de cette xanthine.

Selon l'un ou l'autre des aspects précédents, la xanthine précitée ou l'un de ses sels ou au moins un extrait végétal le contenant, ou une combinaison des composés et extraits précités, est au moins en partie incorporée dans des phases lamellaires lipidiques hydratées ou dans des vésicules de type liposome.

Dans le cadre de ce procédé, la xanthine précitée ou l'un de ses sels tels que précédemment définis, ou au moins un extrait végétal la contenant, ou une combinaison des xanthines et extraits précités, peut être introduit dans un excipient, véhicule ou support cosmétiquement acceptable:

Par l'expression "incorporé au moins en partie dans des phases lamellaires lipidiques hydratées ou dans des vésicules type liposome", on entend, dans la présente description et les revendications, que le composé de Formule (I) (ou l'extrait végétal le contenant) est combiné à des phases lamellaires lipidiques hydratées ou avec des liposomes, quelle que soit la forme de cette combinaison. Cependant, une combinaison préférée réside dans l'incorporation ou l'encapsulation du composé de Formule (I) (ou de l'extrait végétal le contenant) dans des phases lamellaires lipidiques hydratées ou dans des vésicules de type liposome. Mais il n'est pas nécessaire que la totalité soit incorporée ou encapsulée pour obtenir l'effet de pigmentation recherché selon l'invention.

Le terme "lipidique" dans l'expression "phase lamellaire lipidique" couvre toutes les substances comprenant une chaîne carbonée dite grasse, comportant généralement plus de 5 atomes de carbone, cette substance étant habituellement dénommée "lipide".

Selon l'invention, on utilise à titre de lipides, pour former soit les phases lamellaires lipidiques, soit les vésicules type liposome, des lipides amphiphiles, c'est-à-dire constitués de molécules possédant un groupe hudrophile indifféremment ionique ou non ionique et un groupe lipophile, ces lipides amphiphiles étant susceptibles de former des phases lamellaires lipidiques ou des vésicules type liposome en présence d'une phase aqueuse.

En particulier, parmi ces lipides, on peut citer : les phospholipides, les phosphoaminolipides, les glycolipides, les alcools gras polyoxyéthylénés, les esters de polyols éventuellement polyoxyéthylénés. De telles substances sont par exemple constituées par une lécithine d'oeuf ou de soja éventuellement hydrogénée, une phosphatidylcholine, une phosphatidylsérine, une sphingomyéline, un cérébroside ou un stéarate de polyglycoloxyéthyléné.

L'incorporation dans des phases lamellaires lipidiques hydratées ou dans des liposomes des composés utilisés conformément à la présente invention peut être réalisée selon des techniques de préparation connues, décrites par exemple dans le document FR-A-2 521 565, et éventuellement en combinaison avec le document FR-A-2 534 487.

Selon la présente invention, on s'est aperçu de manière totalement inattendue que toutes les xanthines de formule I précitée ne possèdent pas des activités équivalentes, en particulier sur la pigmentation de la peau ou des cheveux.

De telles xanthines substituées en 1 et/ou en 3 sont beaucoup plus actives que les autres, en particulier que certaines substituées également en 7 telles que la caféine (1,3,7-triméthylxanthine).

En outre, l'encapsulation en phases lamellaires lipidiques hydratées ou en liposomes potentialise radicalement l'activité de ces xanthines substituées en 1 et/ou en 3 alors que celle des xanthines substituées en 7 reste très sensiblement plus faible. Cette potentialisation a été observée par des essais réalisés in vivo.

Ainsi, la découverte de la forte activité pigmentante des xanthines substituées en 1 et/ou en 3 est tout à fait surprenante pour un homme de l'art.

D'autres buts, caractéristiques et avantages de l'invention apparaîtront clairement à la lumière de la description explicative qui va suivre faite en référence à plusieurs exemples donnés simplement à titre d'illustration et qui ne sauraient donc en aucune façon limiter la portée de l'invention.

Dans les exemples, les pourcentages sont donnés en poids, sauf indication contraire.

## Exemple 1

### Préparation de liposomes contenant une xanthine selon l'invention

Les xanthines ayant un caractère hydrophobe sont de préférence incorporées dans la phase lipidique.

Pour ce faire, on procède, par exemple, comme suit :

on dissout 0,2 g de 3-isobutyl-1-méthylxanthine (IBMX), 3,6 g de lécithine de soja, 0,4 g de $\beta$-sitostérol dans 25 ml de dichlorométhane. On évapore cette solution organique au ballon rotatif à 45°C sous pression réduite de manière à obtenir un film lipidique déposé sur la paroi intérieure du ballon.

Le film obtenu est repris par 95,8 g d'eau distillée ou de solution tampon pour obtenir une suspension de vésicules lipidiques. On procède à une sonication à 100 W pendant 10 min à 4°C à la suite de quoi on obtient des liposomes présentant une taille moyenne vésiculaire de 142 nm, encapsulant au moins en partie l'IBMX.

On peut procéder de même avec d'autres xanthines selon la présente invention telles que précédemment définies.

Dans l'exemple 2 suivant, on procède à la mise en évidence de la stimulation de la mélanogénèse in vivo chez l'animal.

## Exemple 2

### Mise en évidence de l'augmentation de la mélanogénèse chez l'animal avec l'IBMX

On utilise des cobayes tricolores adultes répartis en 4 lots de 3 ou 4 cobayes. On procède de la manière suivante :

Avant et pendant l'expérimentation, les flancs droit et gauche des cobayes sont soigneusement rasés, chaque jour durant les 5 premiers jours (période d'exposition aux UV) puis tous les 2 jours jusqu'à la fin de l'étude.

Pour chaque animal, on détermine sur chaque flanc des taches comparablement pigmentées, d'aspect le plus souvent marron clair. Sur l'un des deux flancs tiré au hasard au début de l'expérimentation, on applique 10 min avant chaque exposition aux rayons ultraviolets 0,05 g environ de produit à tester ou de produit témoin, selon le lot, l'autre flanc étant exposé "nu" à titre de contrôle.

Durant les 12 jours suivant la cinquième exposition aux UV, les applications sont continuées dans les mêmes conditions.

L'exposition au rayonnement ultraviolet est effectuée au moyen d'un simulateur solaire délivrant 86 % d'UVA et 14 % d'UVB à raison de 5 min le premier jour, 10 min le deuxième jour, 15 min le troisième jour et 20 min les quatrième et cinquième jours.

12 jours après la dernière exposition on prélève un fragment de peau sur le flanc non traité mais exposé, ainsi que sur l'autre flanc traité et exposé.

On effectue ensuite un examen histologique des fragments cutanés.

Cet examen comporte : d'une part, l'étude de la mélanogénèse par la méthode Argentaffine de Fontana sur des coupes de 4 µm (Techniques d'histologie, professeur Chevreau, édition Maloine, 1977, page 157), d'autre part, l'appréciation de l'épaisseur de l'épiderme sur des coupes de 4 µm colorées selon la méthode Trichromique de Masson (Hématoxyline-Fuchsine Acide ponceau de Xylidine bleue d'aniline).

Pour étudier la mélanogénèse, on examine deux zones tirées au hasard d'une tache pigmentée, dans lesquelles on repère 25 cellules malpigiennes et on compte parmi celles-ci les mélanocytes "activés", c'est-à-dire contenant de la mélanine en amas. On exprime alors l'activation du processus de la mélanogénèse en pourcentage de cellules activées à partir de la moyenne de ces deux valeurs. On effectue également un repérage des formes rhizomiques des mélanocytes.

Sur ces mêmes zones, on examine la quantité de mélanine dans les autres couches de l'épiderme et on apprécie globalement cette quantité suivant une échelle à 5 valeurs variant de 0 à 4 selon que la quantité de mélanine formée est, respectivement, nulle, faible, moyenne, importante ou très importante.

Enfin, on mesure sur ces zones l'épaisseur de l'épiderme au moyen d'un micromètre sur microscope binoculaire.

On a regroupé au tableau I les résultats de l'étude histologique donnant le pourcentage d'activation et permettant d'apprécier les variations de la quantité de mélanine formée (valeurs moyennes selon l'échelle définie ci-dessus), de l'épaisseur de l'épiderme (exprimée en µm).

Les produits à tester sont les suivants :

- produit "$I_1$" : suspension gélifiée de liposomes
  contenant 0,1% d'IBMX, préparée selon l'exemple 9
- produit "$I_2$" : solution gélifiée d'IBMX

| | |
|---|---|
| IBMX | 0,1 g |
| éthanol | 30,0 g |
| eau bidistillée | 19,9 g |
| gel de Carbopol 940® à 1% | 50,0 g |

- produit "$A_1$" : suspension gélifiée de liposomes témoins
  (liposomes "vides")

| Composition pour 100 g : | |
|---|---|
| lécithine de soja | 1,8 g |
| β-sitostérol | 0,2 g |
| eau distillée | 48,0 g |
| gel de Carbopol 940® à 1% | 50,0 g |

- produit "$A_2$" : gel de Carbopol® éthanolique

| | |
|---|---|
| éthanol | 30,0 g |
| eau distillée | 20,0 g |
| gel de Carbopol 940® à 1% | 50,0 g |

Les résultats indiqués au tableau I confirment l'activité marquée de l'IBMX, et plus particulièrement celle de l'IBMX encapsulé en liposomes conforme à l'invention, sur la mélanogénèse sur animal.

Tableau I

| | Mélanocytes activés % | | Quantité de mélanine formée | | Epaisseur de l'épiderme (µm) | | Mélanocytes présentant un aspect rhizomique % | |
|---|---|---|---|---|---|---|---|---|
| | FC | FT | FC | FT | FC | FT | FC | FT |
| $I_1$ (0,1% IBMX en liposomes dans gel) | 55,71 | 90,00 | 1,50 | 3,50 | 9,92 | 13,29 | 0 | 100 |
| $I_2$ (0,1% IBMX dans gel) | 35,14 | 77,71 | 0,64 | 2,21 | 8,43 | 9,50 | 0 | 10 |
| $A_1$ (liposomes "vides" dans gel) | 36,67 | 87,00 | 0,91 | 2,50 | 7,75 | 8,50 | 0 | 0 |
| $A_2$ (gel) | 32,86 | 78,86 | 0,43 | 2,14 | 9,36 | 8,43 | 0 | 0 |

FC : flanc contrôle, non traité

FT : flanc traité

On remarque à partir du tableau I que tous les résultats obtenus avec l'IBMX en liposome ($I_1$) sont supérieurs à ceux obtenus pour les autres produits testés. Ceci est particulièrement marquant en ce qui concerne la quantité de mélanine formée et beaucoup plus encore en ce qui concerne l'aspect rhizomique des mélanocytes. Il est rappelé que lorsque le mélanocyte est activé, et qu'il produit de la mélanine sous forme de mélanosomes, la forme du mélanocyte se modifie de manière à former des sortes de

"tentacules", appelées généralement "dendrites", l'aspect du mélanocyte est dit "rhizomique". Les pigments mélaniques sont alors acheminés par les dendrites vers les couches supérieures de l'épiderme. L'aspect "rhizomique" est donc un excellent critère de mise en évidence de l'activité de la mélanogénèse. Par contre, au repos, les mélanocytes perdent cet aspect pour reprendre une forme plus régulière. On observe donc en outre, d'après le tableau I que l'IBMX sous forme libre dans un gel éthanolique ($I_2$) est également plus actif que les deux produits témoins ($A_1$) et ($A_2$).

L'épaisseur de l'épiderme qui est à mettre en relation avec la défense de l'organisme contre le rayonnement ultraviolet est aussi plus importante chez les animaux traités à l'IBMX en liposome selon l'invention (I).

## Exemple 3

### Mise en évidence de l'augmentation de la mélanogénèse chez l'animal avec différentes xanthines

On procède selon le protocole décrit à l'exemple 2, avec les produits suivants :

- produit "$I_3$" : solution gélifiée de 3-méthylxanthine à 0,05%
  préparée comme le produit $I_2$ de l'exemple 2
- produit "$I_4$" : solution gélifiée de 1-méthylxanthine à 0,05%
  préparée comme le produit $I_2$ de l'exemple 2
- produit "$I_5$" : solution gélifiée de 3,7-diméthylxanthine à 0,05%
  préparée comme le produit $I_2$ de l'exemple 2
- produit "$I_6$" : suspension gélifiée de liposomes contenant 0,1% de 1,3-diméthylxanthine
  préparée selon l'exemple 9

Les résultats figurent au tableau II.

Tableau II

| | Mélanocytes activés % | | Quantité de mélanine formée | | Epaisseur de l'épiderme (µm) | | Mélanocytes présentant un aspect rhizomique % | |
|---|---|---|---|---|---|---|---|---|
| | FC | FT | FC | FT | FC | FT | FC | FT |
| $I_3$ | 26,63 | 85,91 | 0,16 | 2,22 | 7,21 | 10,07 | 0 | 80 |
| $I_4$ | 27,70 | 82,10 | 0,06 | 1,87 | 8,24 | 11,19 | 0 | 60 |
| $I_5$ | 32,14 | 86,50 | 0,20 | 1,95 | 8,80 | 10,32 | 0 | 30 |
| $I_6$ | 35,60 | 73,70 | 0,69 | 2,09 | 9,73 | 11,23 | 0 | 30 |

FC : flanc contrôle non traité
FT : flanc traité

Il apparaît clairement d'après les résultats expérimentaux obtenus in vivo pour les produits $I_1$ à $I_6$ une activité très significative sur la stimulation de la pigmentation cutanée, par le biais d'une action sur les mélanocytes. On observera que les produits $I_3$ et $I_4$ conduisent aux meilleurs résultats. On notera ici encore

un épaississement de l'épiderme avec les produits de l'invention, ce qui a tendance à favoriser les défenses naturelles contre le rayonnement solaire.

On donnera ci-après divers exemples de formulation de compositions cosmétiques ou pharmaceutiques, notamment dermatologiques ayant une activité dans le traitement des désordres de la pigmentation cutanée.

Exemple 4

Gel aqueux bronzant pour le visage

| | |
|---|---|
| IBMX | 0,1 g |
| Ethanol | 30,- g |
| Eau distillée | 19,9 g |
| Gel Carbopol® 940 à 1 % | qsp 100 g |

Exemple 5

Crème solaire bronzante

| | |
|---|---|
| 3-méthylxanthine | 0,05 g |
| Stéarate d'isocétyle | 8,- g |
| Huile d'arachide hydrogénée | 10,- g |
| Huile de lanoline | 3,5 g |
| Alcool cétylique | 5,- g |
| Alcool stéarylique | 2,5 g |
| Huile de vaseline légère | 10,- g |
| Monoester phosphorique de l'alcool cétylique OE neutralisé | 3,- g |

Cette phase est émulsionnée avec une phase aqueuse qsp 100 g contenant :

| | |
|---|---|
| Pantothénol | 0,1 g |
| Conservateurs | 0,2 g |
| Filtre solaire hydrosoluble | 5,- g |

Exemple 6

Lotion pour renforcer la protection solaire naturelle

| | |
|---|---|
| Alcool | 42,5 g |
| Propylèneglycol | 3,- g |
| Menthol | 0,05 g |
| Hydroxypropylméthylcellulose | 1,5 g |
| 1-méthylxanthine (poids sec) | 0,2 g |
| Excipients aqueux parfumés | qsp 100 g |

Cette lotion est appliquée localement, de préférence deux fois par jour, quotidiennement pendant 3 à 8 jours précédant les expositions prolongées au soleil.

Exemple 7

Lotion tonique capillaire contre les cheveux gris

| | |
|---|---|
| IBMX | 0,2 g |
| Alcool | 60,- g |
| Eau | 37,- g |
| Excipients parfumés | qsp 100 g |

Cette lotion peut être appliquée sur les cheveux et le cuir chevelu deux fois par jour par cures de deux mois.

Exemple 8

Crème dermatologique pour le traitement du vitiligo

| | |
|---|---|
| IBMX | 0,5 g |
| Lécithine de soja | 1,5 g |
| Perhydrosqualène | 83,- g |

Ces constituants sont chauffés au bain-marie à 70 °C pendant 15 min. 50 g de cette phase grasse obtenue sont repris par 225 ml d'eau bidistillée. On agite au moyen d'un agitateur Raynerie, puis on gélifie avec 125 g d'un gel de Carbopol® 940 de la composition figurant à l'exemple 9 ci-dessous. On obtient ainsi une crème qui est utilisée une à deux fois par jour en application locale sur les zones dépigmentées.

Exemple 9

Préparation d'un gel à partir d'une suspension liposomique de IBMX

A 50 g de la suspension homogénéisée obtenue à l'exemple 1, on mélange 50 g du gel de Carbopol® 940 de composition suivante:

| | |
|---|---|
| Carbopol 940® | 2,- g |
| Nipagin M® | 0,35 g |
| Nipasol® | 0,02 g |
| Dissolvine® | 0,005 g |
| Triéthanolamine | 2,50 g |
| Glycérine | 3,- g |
| Eau distillée | qsp 100 g |

Ce gel peut être utilisé par applications locales, par exemple pour renforcer la protection solaire naturelle.

Exemple 10

Préparation d'une composition cosmétique pigmentante à base d'un extrait naturel contenant de la théobromine

Préparation de l'extrait de cacao

L'extrait de cacao est obtenu à partir de graines de cacao, selon une méthode d'extraction basée sur celle décrite dans le document US-A-2 275 835, et en particulier sur celle des exemples. Cet extrait contient un mélange de théobromine et de caféine.

Composition cosmétique :

| | |
|---|---|
| extrait de cacao | 1,0 g |
| éthanol | 29,0 g |
| eau distillée | 20,0 g |
| gel de Carbopol 940® à 1% | 50,0 g |
| | 100,0 g |

Cette composition peut être utilisée comme décrit à l'exemple 9.

Naturellement, l'invention comprend tous les moyens constituant des équivalents techniques des moyens décrits ainsi que leurs diverses combinaisons.

## Revendications

**1.** Utilisation d'une xanthine de Formule (I) suivante :

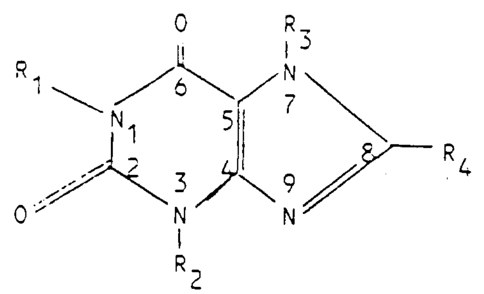

(I)

dans laquelle :

$R_1$ et $R_2$ sont identiques ou différents et représentent chacun un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle;

$R_3$ représente un atome d'hydrogène, un radical alkyle ayant de préférence de 1 à 4 atomes de carbone, un radical benzyle, ledit radical alkyle étant éventuellement substitué par un reste hétérocyclique tel que le reste 4-morpholinyle ou 1-pipéridinyle, ou par un ou plusieurs atomes d'halogène ou groupes hydroxy;

$R_4$ représente un atome d'hydrogène ou d'halogène, un radical alkyle à chaîne droite, ramifiée ou cyclique, ayant de préférence de 1 à 6 atomes de carbone, un radical aryle ou un groupe

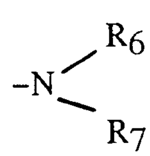

dans lequel $R_6$ et $R_7$ représentent indépendamment un atome d'hydrogène, un radical alkyle ayant de 1 à 6 atomes de carbone, de préférence un radical méthyle ou éthyle, ou bien forment ensemble et avec l'atome d'azote un hétérocycle de préférence monocyclique saturé, lesdits radicaux alkyle ou aryle étant éventuellement substitués par un ou plusieurs atomes d'halogène ou groupes hydroxy, méthoxy, carbométhoxy ou amino;

ou l'utilisation d'un sel pharmaceutiquement acceptable de cette xanthine, ou celle d'un extrait

végétal contenant une telle xanthine;

pour la préparation d'une composition pharmaceutique, notamment dermatologique, destinée à favoriser la pigmentation de la peau ou des cheveux ou à renforcer les défenses naturelles de la peau contre les rayonnements solaires.

**2.** Utilisation d'une xanthine de Formule (I) suivante :

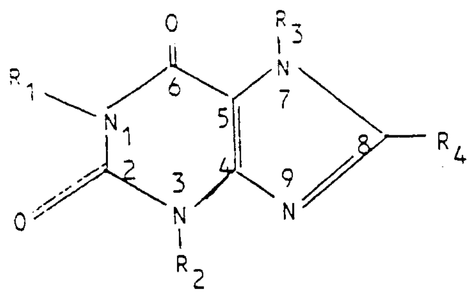

(I)

dans laquelle :

$R_1$ et $R_2$ sont identiques ou différents et représentent chacun un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle;

$R_3$ représente un atome d'hydrogène, un radical alkyle ayant de préférence de 1 à 4 atomes de carbone, un radical benzyle, ledit radical alkyle étant éventuellement substitué par un reste hétérocyclique tel que le reste 4-morpholinyle ou 1-pipéridinyle, ou par un ou plusieurs atomes d'halogène ou groupes hydroxy;

$R_4$ représente un atome d'hydrogène ou d'halogène, un radical alkyle à chaîne droite, ramifiée ou cyclique, ayant de préférence de 1 à 6 atomes de carbone, un radical aryle ou un groupe

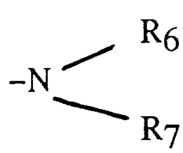

dans lequel $R_6$ et $R_7$ représentent indépendamment un atome d'hydrogène, un radical alkyle ayant de 1 à 6 atomes de carbone, de préférence un radical méthyle ou éthyle, ou bien forment ensemble et avec l'atome d'azote un hétérocycle de préférence monocyclique saturé, lesdits radicaux alkyle ou aryle étant éventuellement substitués par un ou plusieurs atomes d'halogène ou groupes hydroxy, méthoxy, carbométhoxy ou amino;

ou l'utilisation d'un sel cosmétiquement acceptable de cette xanthine, ou celle d'un extrait végétal contenant une telle xanthine,

comme agent cosmétique de pigmentation de la peau ou des cheveux ou de renforcement des défenses naturelles de la peau contre les rayonnements solaires.

**3.** Utilisation selon l'une des revendications 1 ou 2, caractérisée en ce que ladite xanthine de Formule (I) précitée ou l'un de ses sels cosmétiquement ou pharmaceutiquement acceptables, ou l'extrait végétal la contenant, ou la combinaison de ladite xanthine et extrait précité, est au moins en partie incorporée dans des phases lamellaires lipidiques hydratées ou dans des vésicules de type liposomes.

**4.** Utilisation selon l'une quelconque des revendications 1 à 3, caractérisée en ce que la xanthine utilisée est une xanthine de Formule (I) précitée dans laquelle :

$R_1$ représente un atome d'hydrogène ou un radical méthyle, éthyle, propyle, butyle, isobutyle, pentyle, isopentyle, 2-propényle, 2-propynyle, benzyle, hydroxyméthyle, 2-hydroxyéthyle, 2-hydroxypropyle ou 3-hydroxypropyle,
$R_2$ représente un atome d'hydrogène ou un radical méthyle, éthyle, propyle, isopropyle, butyle, isobutyle, 2-propényle, 2-méthyl-2-propényle, cyclohexyle, benzyle ou 2,3-dibromopropyle,
$R_3$ représente un atome d'hydrogène ou un radical méthyle, chlorométhyle, hydroxyméthyle, 2-chloroéthyle, 4-morpholinylméthyle ou benzyle,
$R_4$ représente un atome d'hydrogène ou de brome, o un radical méthyle, éthyle, cyclopropyle, cyclopentyle, cyclohexyle, tertiobutyle, 2-carbométhoxyéthyle, hydroxyméthyle, méthoxyméthyle, méthylamino, 1-pipéridinyle.

**5.** Utilisation selon l'une des revendications 1 à 4, caractérisée en ce que la xanthine de Formule (I) précitée est choisie parmi le groupe consistant de :
Xanthine,
1,8-diméthyl-3-isobutyl-xanthine,
7-benzyl-1,8-diméthyl-3-isobutyl-xanthine,
1-(2-hydroxyéthyl)-3-isobutyl-8-méthyl-xanthine,
1,8-diméthyl-3-(2-méthyl-2-propényl)-xanthine,
3-(2,3-dibromopropyl)-1-éthyl-8-méthyl-xanthine,
1,3-di(2-propényl)-8-méthyl-xanthine,
7-benzyl-8-bromo-3-isobutyl-1-méthyl-xanthine,
8-éthyl-1-(2-hydroxyéthyl)-3-isobutyl-xanthine,
8-hydroxyméthyl-3-isobutyl-1-méthyl-xanthine,
3-isobutyl-8-méthoxyméthyl-1-méthyl-xanthine,
8-(2-carbométhoxyéthyle)-3-isobutyl-1-méthyl-xanthine,
3-isobutyl-1-méthyl-8-tertio-butyl-xanthine,
3-isobutyl-1-méthyl-8-méthylamino-xanthine,
1,3-dipropyl-8-(1-pipéridinyl)-xanthine,
8-cyclopropyl-1,3-dipropyl-xanthine,
8-cyclopentyl-1,3-dipropyl-xanthine,
8-cyclohexyl-1,3-dipropyl-xanthine,
1,3-diméthyl-8-phényl-xanthine,
1,3-dipropyl-8-phényl-xanthine,
8-(2-amino-4-chlorophényl)-1,3-dipropyl-xanthine,
1,3-diéthyl-7-méthyl-xanthine,
1,3-dipropyl-7-méthyl-xanthine,
7-chlorométhyl-1,3-diméthyl-xanthine,
1,3-diméthyl-7-hydroxyméthyl-xanthine,
1,3-diméthyl-7-(4-morpholinylméthyl)-xanthine,
1,3,7-triéthyl-xanthine,
3,7-diéthyl-1-(2-propynyl)-xanthine,
7-(2-chloroéthyl)-1,3-diméthyl-xanthine.

**6.** Utilisation selon la revendication 5, caractérisée en ce que la xanthine est choisie parmi le groupe consistant de :
1,8-diméthyl-3-isobutyl-xanthine,
8-(2-carbométhoxyéthyl)-3-isobutyl-xanthine, et
3-isobutyl-1-méthyl-8-tertiobutyl-xanthine.

**7.** Utilisation selon l'une quelconque des revendications 1 à 4, caractérisée en ce que, dans la Formule (I) précitée, $R_3$ et R4 représentent chacun un atome d'hydrogène, et
$R_1$ et $R_2$ représentent chacun, indépendamment l'un de l'autre, un atome d'hydrogène ou un radical tel que précédemment défini.

**8.** Utilisation selon la revendication 7, caractérisée en ce que la xanthine précitée est choisie parmi le groupe consistant de :
3-isobutyl-1-méthyl-xanthine,
3-benzyl-1-méthyl-xanthine,

3-isopentyl-1-méthyl-xanthine,
1-méthyl-3-(2-propényl)-xanthine,
1-méthyl-3-(2-méthyl-2-propényl)-xanthine,
3-butyl-1-méthyl-xanthine,
1,3-diéthyl-xanthine,
1-éthyl-3 propyl-xanthine,
3-butyl-1-éthyl-xanthine,
1-éthyl-3-(2-méthyl-2-propényl)-xanthine,
3-cyclohexyl-1-éthyl-xanthine,
1-(2-hydroxyéthyl)-3-isobutyl-xanthine,
1,3-dipropyl-xanthine,
1,3-di(2-propényl)-xanthine,
1,3-dibutyl-xanthine,
3-éthyl-1-pentyl-xanthine,
1-benzyl-3-isobutyl-xanthine.

**9.** Utilisation selon la revendication 8, caractérisée en ce que la xanthine est choisie parmi le groupe consistant de :
3-isobutyl-1-méthyl-xanthine,
3-benzyl-1-méthyl-xanthine,
1,3-diéthyl-xanthine;
1-éthyl-3-propyl-xanthine,
3-butyl-1-éthyl-xanthine,
1,3-dipropyl-xanthine,
1,3-dibutyl-xanthine.

**10.** Utilisation selon l'une quelconque des revendications 1 à 3, caractérisée en ce qu'on utilise la 1,3-diméthyl-xanthine ou la 3,7-diméthyl-xanthine.

**11.** Utilisation selon l'une quelconque des revendications 1 à 3, caractérisée en ce qu'on utilise la 3-isobutyl-1-méthyl-xanthine.

**12.** Utilisation selon l'une quelconque des revendications 1 à 3, caractérisée en ce que, dans la Formule (I) précitée, $R_3$ et $R_4$ représentent chacun un atome d'hydrogène, et $R_1$ et $R_2$ représentent un atome d'hydrogène ou un radical tel que précédemment défini, au moins $R_1$ ou $R_2$ représentant un atome d'hydrogène.

**13.** Utilisation selon la revendication 12, caractérisée en ce que $R_2$, $R_3$ et $R_4$ représentent chacun un atome d'hydrogène, et $R_1$ représente un radical alkyle ayant de préférence de 1 à 4 atomes de carbone, notamment un radical méthyle, éthyle, propyle, ou butyle, de préférence un radical méthyle.

**14.** Utilisation selon la revendication 12, caractérisée en ce que, dans la Formule (I) précitée, $R_1$, $R_3$,et $R_4$ représentent chacun un atome d'hydrogène, et $R_2$ représente un radical tel que précédemment défini, en particulier un radical alkyle ayant de préférence de 1 à 4 atomes de carbone.

**15.** Utilisation selon la revendication 14, caractérisée en ce que $R_2$ représente un radical méthyle, propyle, butyle ou isobutyle.

**16.** Utilisation selon l'une quelconque des revendications 1 à 15, caractérisée en ce que le sel pharmaceutiquement ou cosmétiquement acceptable est un sel alcalin tel qu'un sel de sodium, de potassium ou d'ammonium, ou un sel de base organique tel que l'éthanolamine, la diéthanolamine, l'éthylènediamine, l'isopropylamine, la triéthylamine, l'octadécylamine ou la choline, ou un sel d'acide aminé tel que la glycine ou la lysine.

**17.** Utilisation selon l'une quelconque des revendications 1 à 16, caractérisée en ce que l'on utilise au moins une xanthine de Formule (I) ou l'un de ses sels tel que précédemment définis, en combinaison avec au moins un extrait végétal précité.

**18.** Utilisation selon l'une quelconque des revendications 1 à 17, caractérisée en ce que la concentration totale en composés de Formule (I) précitée, ou de leurs sels, ou des extraits végétaux les contenant est comprise entre 0,001% et 10% en poids, de préférence entre 0,01% et 1% en poids, par rapport au poids total de la composition.

**19.** Utilisation d'une xanthine ou l'un de ses sels pharmaceutiquement acceptables, de Formule (Ia) :

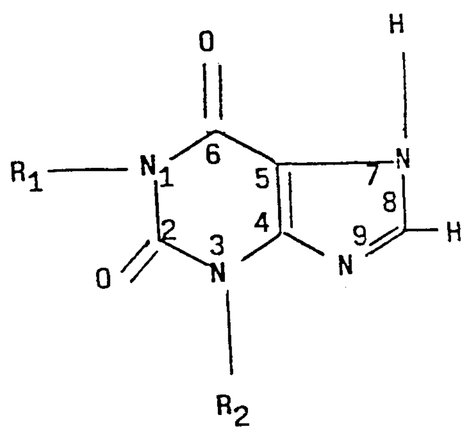

(Ia)

dans laquelle :
$R_1$ et $R_2$ sont différents et représentent chacun un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle ; l'un parmi $R_1$ ou $R_2$ étant un atome d'hydrogène,

ou au moins un extrait végétal contenant une xanthine de formule (Ia), ou la combinaison des composé et extraits ci-dessus,

dans un excipient, véhicule ou support pharmaceutiquement acceptable;

pour la préparation d'une composition pharmaceutique, notamment dermatologique, destinée à favoriser la pigmentation de la peau ou des cheveux ou à renforcer les défenses naturelles de la peau contre les rayonnements solaires.

**20.** Utilisation d'une xanthine ou l'un de ses sels cosmétiquement acceptables, de Formule (Ia) :

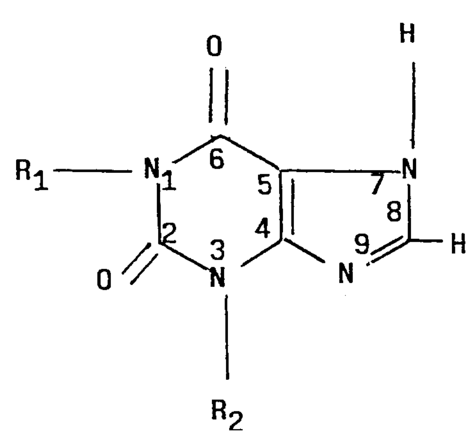

(Ia)

dans laquelle :
$R_1$ et $R_2$ sont différents et représentent chacun un atome d'hydrogène, un radical alkyle à chaîne

droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle ; l'un parmi $R_1$ ou $R_2$ étant un atome d'hydrogène,

ou au moins un extrait végétal contenant une xanthine de formule (Ia), ou la combinaison des composés et extraits ci-dessus,

dans un excipient, véhicule ou support cosmétiquement acceptable;

comme agent cosmétique de pigmentation de la peau ou des cheveux ou de renforcement des défenses naturelles de la peau contre les rayonnements solaires.

**21.** Utilisation selon la revendication 19 ou 20, caractérisée en ce que la xanthine précitée ou l'extrait végétal la contenant, ou la combinaison de la xanthine et de l'extrait précité est au moins en partie incorporée dans des phases lamellaires lipidiques hydratées ou dans des vésicules de type liposomes.

**22.** Utilisation selon l'une quelconque des revendications 19 à 21, caractérisée en ce que $R_2$ représente un atome d'hydrogène et $R_1$ représente un radical alkyle ayant de préférence de 1 à 4 atomes de carbone, notamment un radical méthyle, éthyle, propyl, ou butyle, de préférence un radical méthyle.

**23.** Utilisation selon l'une quelconque des revendications 19 à 21, caractérisée en ce que $R_1$ représente un atome d'hydrogène et $R_2$ représente un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyl ayant de préférence de 7 à 12 atomes de carbone, lesdits radicaux alkyle, alcenyle et aralkyle étant éventuellement substitués par un ou plusieurs atomes d'halogène ou par un ou plusieurs groupes $-OR_5$ dans lequel $R_5$ représente un atome d'hydrogène ou un radical alkyl ayant de 1 à 6 atomes de carbone, de préférence un radical méthyl ou éthyl.

**24.** Utilisation selon la revendication 23, caractérisée en ce que $R_2$ représente un radical alkyle ayant de préférence de 1 à 4 atomes de carbone, encore mieux un radical méthyle, propyle, butyle ou isobutyle.

**25.** Utilisation selon l'une des revendications 19 à 24, caractérisée en ce que la concentration totale en composés de Formule (Ia) précitée, ou de leurs sels, ou des extraits végétaux les contenant est comprise entre 0,001% et 10% en poids, de préférence entre 0,01% et 1% en poids, par rapport au poids total de la composition.

**26.** Composition cosmétique, ou pharmaceutique, notamment dermatologique, notamment destinée à favoriser la pigmentation de la peau ou des cheveux,

caractérisée en ce qu'elle comprend au moins une xanthine de Formule (Ia) ou de l'un de ses sels cosmétiquement ou pharmaceutiquement acceptables, de Formule (Ia) :

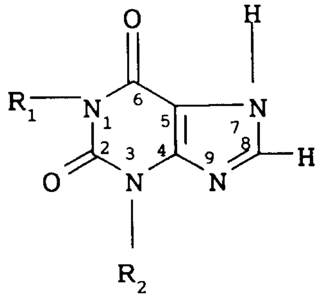

(Ia)

dans laquelle :

$R_1$ et $R_2$ sont différents et représentent chacun un atome d'hydrogène, un radical alkyle à chaîne droite, ramifiée ou cyclique ayant de préférence de 1 à 8 atomes de carbone, un radical alcényle à

chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle ; l'un parmi $R_1$ ou $R_2$ étant un atome d'hydrogène,

ou au moins un extrait végétal contenant une xanthine de formule (Ia), ou la combinaison des composé et extraits ci-dessus, dans un excipient, véhicule ou support cosmétiquement ou pharmaceutiquement acceptable.

**27.** Composition cosmétique, ou pharmaceutique, notamment dermatologique, notamment destinée à favoriser la pigmentation de la peau ou des cheveux, caractérisée en ce qu'elle comprend une quantité efficace pour la production de la pigmentation d'au moins une xanthine choisie parmi le groupe consistant de :

1,8-diméthyl-3-isobutylxanthine,
7-benzyl-1,8-diméthyl-3-isobutylxanthine,
1-(2-hydroxyéthyl)-3-isobutyl-8-méthylxanthine
1,8-diméthyl-3-(2-méthyl-2-propényl)xanthine,
3-(2,3-dibromopropyl)-1-éthyl-8-méthylxanthine,
1,3-di(2-propényl)-8-méthylxanthine,
7-benzyl-8-bromo-3-isobutyl-1-méthylxanthine,
8-éthyl-1-(2-hydroxyéthyl)-3-isobutylxanthine,
8-hydroxyméthyl-3-isobutyl-1-méthylxanthine,
3-isobutyl-8-méthoxyméthyl-1-méthylxanthine,
8-(2-carbométhoxyéthyl)-3-isobutyl-1-méthylxanthine,
3-isobutyl-1-méthyl-8-tert-butylxanthine,
3-isobutyl-1-méthyl-8-méthylaminoxanthine,
1,3-dipropyl-8(pipéridin-1-yl)xanthine,
8-cyclopropyl-1,3-dipropylxanthine,
8-cyclopentyl-1,3-dipropylxanthine,
8-cyclohexyl-1,3-dipropylxanthine,
1,3-diméthyl-8-phénylxanthine,
1,3-dipropyl-8-phénylxanthine,
8-(2-amino-4-chlorophényl)-1,3-dipropylxanthine,
1,3-diéthyl-7-méthylxanthine,
1,3-dipropyl-7-méthylxanthine,
7-chlorométhyl-1,3-diméthylxanthine,
1,3-diméthyl-7-hydroxyméthylxanthine,
1,3-diméthyl-7-(morpholin-4-yl-méthyl)xanthine,
1,3,7-triéthylxanthine,
3,7-diéthyl-1-(2-propényl)xanthine,
7-(2-chloroéthyl)-1,3-diméthylxanthine,
3-benzyl-1-méthylxanthine,
3-isopentyl-1-méthylxanthine,
1-méthyl-3-(2-propényl)xanthine,
1-méthyl-3-(2-méthyl-2-propényl)xanthine,
3-butyl-1-méthylxanthine,
1,3-diéthylxanthine,
1-éthyl-3-propylxanthine,
3-butyl-1-éthylxanthine,
1-éthyl-3-(2-méthyl-2-propényl)xanthine,
3-cyclohéxyl-1-éthylxanthine,
1-(2-hydroxyéthyl)-3-isobutylxanthine,
1,3-dipropylxanthine,
1,3-di-(2-propénylxanthine,
1,3-dibutylxanthine,
3-éthyl-1-penthylxanthine,
1-benzyl-3-isobutylxanthine,

1-méthylxanthine,
1-éthylxanthine,
1-propylxanthine,
1-butylxanthine,
3-méthylxanthine,
3-propylxanthine,
3-butylxanthine, et
3-isobutylxanthine,

ou un sel pharmaceutiquement ou cosmétiquement acceptable de cette xanthine, au moins un extrait végétal contenant une telle xanthine, ou une combinaison des composés et extraits ci-dessus, dans un excipient, véhicule ou support cosmétiquement ou pharmaceutiquement acceptable.

**28.** Composition selon l'une des revendications 26 ou 27, caractérisée en ce que la concentration totale en composés de Formule (Ia) précitée, ou de leurs sels, ou des extraits végétaux précités les contenant, est comprise entre 0,001% et 10% en poids, de préférence entre 0,01% et 1% en poids, par rapport au poids total de la composition finale.

**29.** Composition selon l'une des revendications 26 à 28, caractérisée en ce qu'elle est formulée pour l'application topique, en particulier sous forme de crème, de gel ou de lotion.

**30.** Composition selon l'une des revendications 26 à 29, caractérisée en ce que la xanthine précitée, ou l'un de ses sels cosmétiquement ou pharmaceutiquement acceptables, ou au moins un extrait végétal la contenant, ou une combinaison de ladite xanthine et extraits précités est au moins en partie incorporée dans des phases lamellaires lipidiques hydratées ou dans des vésicules de type liposome.

**31.** Procédé de traitement cosmétique de la peau ou de la chevelure pour promouvoir la pigmentation, ledit procédé comprenant l'application à la peau ou à la chevelure à pigmenter d'une quantité efficace pour produire la pigmentation, d'au moins une xanthine de Formule (I) suivante :

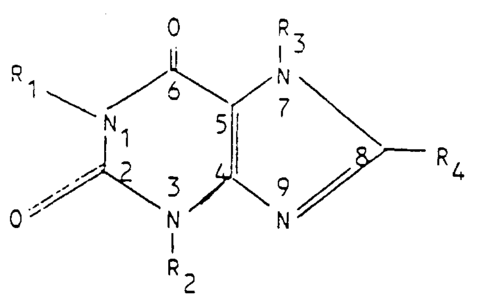

(I)

dans laquelle :

$R_1$ et $R_2$ sont identiques ou différents et représentent chacun un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle;

$R_3$ représente un atome d'hydrogène, un radical alkyle ayant de préférence de 1 à 4 atomes de carbone, un radical benzyle, ledit radical alkyle étant éventuellement substitué par un reste hétérocyclique tel que le reste 4-morpholinyle ou 1-pipéridinyle, ou par un ou plusieurs atomes d'halogène ou groupes hydroxy;

$R_4$ représente un atome d'hydrogène ou d'halogène, un radical alkyle à chaîne droite, ramifiée ou cyclique, ayant de préférence de 1 à 6 atomes de carbone, un radical aryle ou un groupe

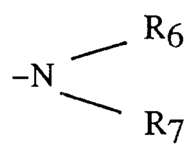

dans lequel $R_6$ et $R_7$ représentent indépendamment un atome d'hydrogène, un radical alkyle ayant de 1 à 6 atomes de carbone, de préférence un radical méthyle ou éthyle, ou bien forment ensemble et avec l'atome d'azote un hétérocycle de préférence monocyclique saturé, lesdits radicaux alkyle ou aryle étant éventuellement substitués par un ou plusieurs atomes d'halogène ou groupes hydroxy, méthoxy, carbométhoxy ou amino;

ou d'un sel cosmétiquement acceptable de cette xanthine, ou d'un extrait végétal contenant une telle xanthine

**32.** Procédé de traitement cosmétique de la peau ou de la chevelure pour promouvoir la pigmentation, ledit procédé comprenant l'application à la peau ou à la chevelure à pigmenter d'une quantité efficace pour produire la pigmentation, d'au moins une xanthine de Formule (Ia) suivante :

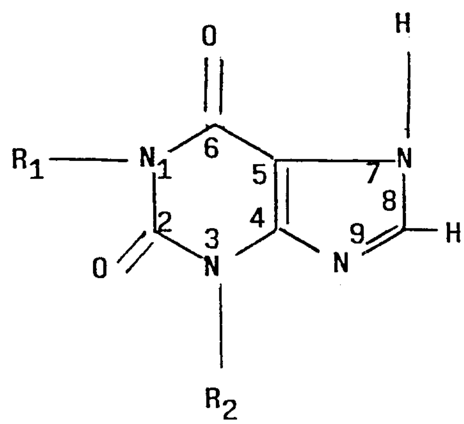

(Ia)

dans laquelle :

$R_1$ et $R_2$ sont différents et représentent chacun un atome d'hydrogène, un radical alkyle à chaîne droite, ramifiée ou cyclique ayant de préférence de 1 à 8 atomes de carbone, un radical alcényle à chaîne droite ou ramifiée ayant de préférence de 2 à 5 atomes de carbone, un radical alcynyle ayant de préférence de 2 à 3 atomes de carbone, un radical aralkyle ayant de préférence de 7 à 12 atomes de carbone, lesdits radicaux alkyle, alcényle et aralkyle étant éventuellement substitués par un ou plusieurs atomes d'halogène ou par un ou plusieurs groupes $-OR_5$ dans lequel $R_5$ représente un atome d'hydrogène ou un radical alkyle ayant de 1 à 6 atomes de carbone, de préférence un radical méthyle ou éthyle ; l'un parmi $R_1$ ou $R_2$ est un atome d'hydrogène,

ou un de ses sels cosmétiquement acceptables.

**33.** Procédé de traitement cosmétique de la peau ou de la chevelure pour promouvoir la pigmentation, ledit procédé comprenant l'application à la peau ou à la chevelure à pigmenter d'une quantité efficace pour produire la pigmentation, d'au moins une xanthine, choisie parmi le groupe consistant de :

1,8-diméthyl-3-isobutylxanthine,
7-benzyl-1,8-diméthyl-3-isobutylxanthine,
1-(2-hydroxyéthyl)-3-isobutyl-8-méthylxanthine,
1,8-diméthyl-3-(2-méthyl-2-propényl)xanthine,
3-(2,3-dibromopropyl)-1-éthyl-8-méthylxanthine,
1,3-di(2-propényl)-8-méthylxanthine,
7-benzyl-8-bromo-3-isobutyl-1-méthylxanthine,
8-éthyl-1-(2-hydroxyéthyl)-3-isobutylxanthine,
8-hydroxyméthyl-3-isobutyl-1-méthylxanthine,
3-isobutyl-8-méthoxyméthyl-1-méthylxanthine,

8-(2-carbométhoxyéthyl)-3-isobutyl-1-méthylxanthine,
3-isobutyl-1-méthyl-8-tert-butylxanthine,
3-isobutyl-1-méthyl-8-méthylaminoxanthine,
1,3-dipropyl-8(pipéridin-1-yl)xanthine,
8-cyclopropyl-1,3-dipropylxanthine,
8-cyclopentyl-1,3-dipropylxanthine,
8-cyclohexyl-1,3-dipropylxanthine,
1,3-diméthyl-8-phénylxanthine,
1,3-dipropyl-8-phénylxanthine,
8-(2-amino-4-chlorophényl)-1,3-dipropylxanthine,
1,3-diéthyl-7-méthylxanthine,
1,3-dipropyl-7-méthylxanthine,
7-chlorométhyl-1,3-diméthylxanthine,
1,3-7-hydroxyméthylxanthine,
1,3-diméthyl-7-morpholin-4-yl-méthyl)xanthine,
1,3,7-triéthylxanthine,
3,7-diéthyl-1-(2-propényl) xanthine,
7-(2-chloroéthyl)-1,3-diméthylxanthine,
3-benzyl-1-méthylxanthine,
3-isopentyl-1-méthylxanthine,
1-méthyl-3-(2-propényl)xanthine,
1-méthyl-3-(2-méthyl-2-propényl)xanthine,
3-butyl-1-méthylxanthine,
1,3-diéthylxanthine,
1-éthyl-3-propylxanthine,
3-butyl-1-éthylxanthine,
1-éthyl-3-(2-méthyl-2-propényl)xanthine,
3-cyclohexyl-1-éthylxanthine,
1-(2-hydroxyéthyl)-3-isobutylxanthine,
1,3-dipropylxanthine,
1,3-di-(2-propényl)xanthine,
1,3-dibutylxanthine,
3-éthyl-1-pentylxanthine,
1-benzyl-3-isobutylxanthine,
1-méthylxanthine,
1-éthylxanthine,
1-propylxanthine,
1-butylxanthine,
3-méthylxanthine,
3-propylxanthine,
3-butylxanthine, et
3-isobutylxanthine,

ou un sel cosmétiquement acceptable de cette xanthine.

**34.** Procédé selon l'une des revendications 31 à 33, caractérisé en ce que la xanthine précitée ou l'un de ses sels cosmétiquement acceptable, ou un extrait végétal la contenant ou une combinaison de ladite xanthine et de l'extrait précité est au moins en partie incorporé dans des phases lamellaires lipidiques hydratées ou dans des vésicules de type liposome.

**35.** Procédé selon l'une des revendications 31 à 34, caractérisé en ce que la xanthine précitée ou l'un de ses sels cosmétiquement acceptables, ou un extrait végétal la contenant ou une combinaison de la xanthine et de l'extrait précité est appliqué à une concentration totale comprise entre 0,001% et 10% en poids, de préférence entre 0,01% et 1% en poids.

## Claims

**1.** Use of a xanthine of formula (I) below:

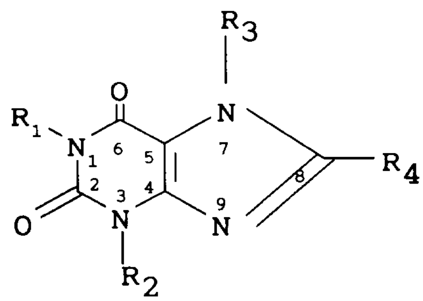

(I)

in which:

$R_1$ and $R_2$ are identical or different and are each a hydrogen atom, an alkyl radical with a linear, branched or cyclic chain, preferably having from 1 to 8 carbon atoms, an alkenyl radical with a linear or branched chain, preferably having from 2 to 5 carbon atoms, an alkynyl radical preferably having from 2 to 3 carbon atoms, an aralkyl radical preferably having from 7 to 12 carbon atoms, said alkyl, alkenyl and aralkyl radicals being optionally substituted by one or more halogen atoms or by one or more groups $-OR_5$, in which $R_5$ is a hydrogen atom or an alkyl radical having from 1 to 6 carbon atoms, preferably a methyl or ethyl radical;

$R_3$ is a hydrogen atom, an alkyl radical preferably having from 1 to 4 carbon atoms, or a benzyl radical, said alkyl radical being optionally substitued by a heterocyclic radical such as the morpholin-4-yl or piperidin-1-yl radical, or by one or more halogen atoms or hydroxyl groups;

$R_4$ is a hydrogen or halogen atom, an alkyl radical with a linear, branched or cyclic chain, preferably having from 1 to 6 carbon atoms, an aryl radical or

a group

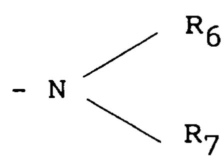

in which $R_6$ and $R_7$ independently are a hydrogen atom or an alkyl radical having from 1 to 6 carbon atoms, preferably a methyl or ethyl radical, or else they form together, with the nitrogen atom, a heterocycle, preferably a saturated monocyclic heterocycle, said alkyl or aryl radicals being optionally substituted by one or more halogen atoms or hydroxyl, methoxy, carbomethoxy or amino groups;

or the use of a pharmaceutically acceptable salt of this xanthine, or that of a plant extract in which such a xanthine is present;

for the preparation of a pharmaceutical composition, especially a dermatological composition, for promoting skin or hair pigmentation or for strengthening the skin's natural defenses against solar radiation.

**2.** Use of a xanthine of formula (I) below:

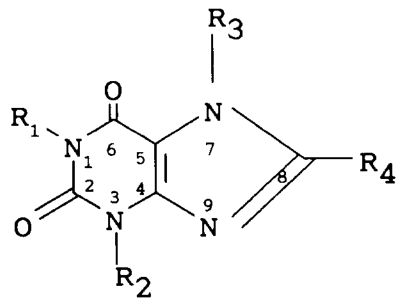

(I)

in which:

$R_1$ and $R_2$ are identical or different and are each a hydrogen atom, an alkyl radical with a linear, branched or cyclic chain, preferably having from 1 to 8 carbon atoms, an alkenyl radical with a linear or branched chain, preferably having from 2 to 5 carbon atoms, an alkynyl radical preferably having from 2 to 3 carbon atoms, an aralkyl radical preferably having from 7 to 12 carbon atoms, said alkyl, alkenyl and aralkyl radicals being optionally substituted by one or more halogen atoms or by one or more groups $-OR_5$, in which $R_5$ is a hydrogen atom or an alkyl radical having from 1 to 6 carbon atoms, preferably a methyl or ethyl radical;

$R_3$ is a hydrogen atom, an alkyl radical preferably having from 1 to 4 carbon atoms, or a benzyl radical, said alkyl radical being optionally substitued by a heterocyclic radical such as the morpholin-4-yl or piperidin-1-yl radical, or by one or more halogen atoms or hydroxyl groups;

$R_4$ is a hydrogen or halogen atom, an alkyl radical with a linear, branched or cyclic chain, preferably having from 1 to 6 carbon atoms, an aryl radical or

a group

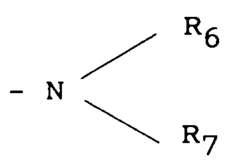

in which $R_6$ and $R_7$ independently are a hydrogen atom, an alkyl radical having from 1 to 6 carbon atoms, preferably a methyl or ethyl radical, or else they form together, with the nitrogen atom, a heterocycle, preferably a saturated monocyclic heterocycle, said alkyl or aryl radicals being optionally substituted by one or more halogen atoms or hydroxyl, methoxy, carbomethoxy or amino groups;

or the use of a cosmetically acceptable salt of this xanthine, or that of a plant extract in which such a xanthine is present;

as a cosmetic agent for the pigmentation of skin or hair or for strengthening the skin's natural defenses against solar radiation.

**3.** Use according to one of claims 1 or 2, characterized in that the xanthine of formula (I) mentioned above, or one of its cosmetically or pharmaceutically acceptable salts, or the plant extract in which it is present, or a combination of the above-mentioned xanthine and extract, is at least partially incorporated into hydrated lipidic lamellar phases or in vesicles of liposome type.

**4.** Use according to one of claims 1 to 3, characterized in that the xanthine used is a xanthine of formula (I) mentioned above in which:

$R_1$ is a hydrogen atom or a methyl, ethyl, propyl, butyl, isobutyl, pentyl, isopentyl, prop-2-enyl, prop-2-ynyl, benzyl, hydroxymethyl, 2-hydroxyethyl, 2-hydroxypropyl or 3-hydroxypropyl radical,

$R_2$ is a hydrogen atom or a methyl, ethyl, propyl, isopropyl, butyl, isobutyl, prop-2-enyl, 2-methylprop-2-enyl, cyclohexyl, benzyl or 2,3-dibromopropyl radical, $R_3$ is a hydrogen atom or a methyl, chloromethyl, hydroxymethyl, 2-chloroethyl, morpholin-4-ylmethyl or benzyl radical,

$R_4$ is a hydrogen or bromine atom or a methyl, ethyl, cyclopropyl, cyclopentyl, cyclohexyl, tert-butyl, 2-carbomethoxyethyl, hydroxymethyl, methoxymethyl, methylamino or piperidin-1-yl radical.

**5.** Use according to one of claims 1 to 4, characterized in that the xanthine of formula (I) mentioned above is selected from the group consisting of:
xanthine,
1,8-dimethyl-3-isobutylxanthine,
7-benzyl-1,8-dimethyl-3-isobutylxanthine,
1-(2-hydroxyethyl)-3-isobutyl-8-methylxanthine,
1,8-dimethyl-3-(2-methylprop-2-enyl)xanthine,
3-(2,3-dibromopropyl)-1-ethyl-8-methylxanthine,
1,3-di(prop-2-enyl)-8-methylxanthine,
7-benzyl-8-bromo-3-isobutyl-1-methylxanthine,
8-ethyl-1-(2-hydroxyethyl)-3-isobutylxanthine,
8-hydroxymethyl-3-isobutyl-1-methylxanthine,
3-isobutyl-8-methoxymethyl-1-methylxanthine,
8-(2-carbomethoxyethyl)-3-isobutyl-1-methylxanthine,
3-isobutyl-1-methyl-8-tert-butylxanthine,
3-isobutyl-1-methyl-8-methylaminoxanthine,
1,3-dipropyl-8-(piperidin-1-yl)xanthine,
8-cyclopropyl-1,3-dipropylxanthine,
8-cyclopentyl-1,3-dipropylxanthine,
8-cyclohexyl-1,3-dipropylxanthine,
1,3-dimethyl-8-phenylxanthine,
1,3-dipropyl-8-phenylxanthine,
8-(2-amino-4-chlorophenyl)-1,3-dipropylxanthine,
1,3-diethyl-7-methylxanthine,
1,3-dipropyl-7-methylxanthine,
7-chloromethyl-1,3-dimethylxanthine,
1,3-dimethyl-7-hydroxymethylxanthine,
1,3-dimethyl-7-(4-morpholinylmethyl)xanthine,
1,3,7-triethylxanthine,
3,7-diethyl-1-(prop-2-ynyl)xanthine,
7-(2-chloroethyl)-1,3-dimethylxanthine.

**6.** Use according to claim 5, characterized in that the xanthine is selected from the group consisting of:
1,8-dimethyl-3-isobutylxanthine,
8-(2-carbomethoxyethyl)-3-isobutylxanthine, and
3-isobutyl-1-methyl-8-tert-butylxanthine.

**7.** Use according to anyone of claims 1 to 4, characterized in that, in formula (I) mentioned above, $R_3$ and $R_4$ are each a hydrogen atom, and
$R_1$ and $R_2$ are each independently of one another a hydrogen atom or a radical as defined above.

**8.** Use according to claim 7, characterized in that the above-mentioned xanthine is selected from the group consisting of:
3-isobutyl-1-methylxanthine,
3-benzyl-1-methylxanthine,
3-isopentyl-1-methylxanthine,
1-methyl-3-(prop-2-enyl)xanthine,
1-methyl-3-(2-methylprop-2-enyl)xanthine,
3-butyl-1-methylxanthine,
1,3-diethylxanthine,
1-ethyl-3 propylxanthine,
3-butyl-1-ethylxanthine,
1-ethyl-3-(2-methylprop-2-enyl)xanthine,
3-cyclohexyl-1-ethylxanthine,
1-(2-hydroxyethyl)-3-isobutylxanthine,
1,3-dipropylxanthine,

1,3-di(prop-2-enyl)xanthine,
1,3-dibutylxanthine,
3-ethyl-1-pentylxanthine,
1-benzyl-3-isobutylxanthine.

**9.** Use according to claim 8, characterized in that the xanthine is selected from the group consisting of:
3-isobutyl-1-methylxanthine,
3-benzyl-1-methylxanthine,
1,3-diethylxanthine,
1-ethyl-3-propylxanthine,
3-butyl-1-ethylxanthine,
1,3-dipropylxanthine,
1,3-dibutylxanthine.

**10.** Use according to anyone of claims 1 to 3, characterized in that 1,3-dimethylxanthine or 3,7-dimethylxanthine is used.

**11.** Use according to anyone of claims 1 to 3, characterized in that 3-isobutyl-1-methylxanthine is used.

**12.** Use according to anyone of claims 1 to 3, characterized in that, in formula (I) mentioned above, $R_3$ and $R_4$ are each a hydrogen atom and $R_1$ and $R_2$ are a hydrogen atom or a radical as defined above, at least $R_1$ or $R_2$ being a hydrogen atom.

**13.** Use according to claim 12, characterized in that $R_2$, $R_3$ and $R_4$ are each a hydrogen atom and $R_1$ is an alkyl radical preferably having from 1 to 4 carbon atoms, especially a methyl, ethyl, propyl or butyl radical and preferably a methyl radical.

**14.** Use according to claim 12, characterized in that, in formula (I) mentioned above, $R_1$, $R_3$ and $R_4$ are each a hydrogen atom and $R_2$ is a radical as defined above, in particular an alkyl radical preferably having from 1 to 4 carbon atoms.

**15.** Use according to claim 14, characterized in that $R_2$ is a methyl, propyl, butyl or isobutyl radical.

**16.** Use according to anyone of claims 1 to 15, characterized in that the pharmaceutically or cosmetically acceptable salt is an alkali metal salt such as a sodium, potassium, or ammonium salt, or a salt of an organic base such as ethanolamine, diethanolamine, ethylenediamine, isopropylamine, triethylamine, octadecylamine or choline, or a salt of an amino acid such as glycine or lysine.

**17.** Use according to anyone of claims 1 to 16, characterized in that at least one xanthine of formula (I) or one of its salts as defined above is used in combination with at least one plant extract mentioned above.

**18.** Use according to anyone of claims 1 to 17, characterized in that the total concentration of compounds of formula (I) mentioned above, or their salts, or plant extracts in which they are present, is between 0.001% and 10% by weight, preferably between 0.01% and 1% by weight, relative to the total weight of the composition.

**19.** Use of a xanthine or one of its pharmaceutically acceptable salts of formula (Ia):

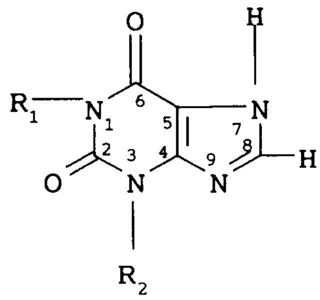

(Ia)

in which:

$R_1$ and $R_2$ are identical or different and are each a hydrogen atom, an alkyl radical with a linear, branched or cyclic chain, preferably having from 1 to 8 carbon atoms, an alkenyl radical with a linear or branched chain, preferably having from 2 to 5 carbon atoms, an alkynyl radical preferably having from 2 to 3 carbon atoms, an aralkyl radical preferably having from 7 to 12 carbon atoms, said alkyl, alkenyl and aralkyl radicals being optionally substituted by one or more halogen atoms or by one or more groups $-OR_5$, in which $R_5$ is a hydrogen atom or an alkyl radical having from 1 to 6 carbon atoms, preferably a methyl or ethyl radical; one of $R_1$ or $R_2$ being a hydrogen atom,

or at least a plant extract containing a xanthine of formula (Ia) or a combination of said compound and extract,

in a pharmaceutically acceptable excipient, vehicle or carrier;

for the preparation of a pharmaceutical, notably dermatological composition, for promoting skin or hair pigmentation or for strengthening the skin's natural defenses against solar radiation.

**20.** Use of a xanthine or one of its cosmetically acceptable salts of formula (Ia):

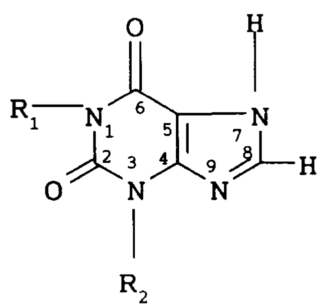

(Ia)

in which:

$R_1$ and $R_2$ are identical or different and are each a hydrogen atom, an alkyl radical with a linear, branched or cyclic chain, preferably having from 1 to 8 carbon atoms, an alkenyl radical with a linear or branched chain, preferably having from 2 to 5 carbon atoms, an alkynyl radical preferably having from 2 to 3 carbon atoms, an aralkyl radical preferably having from 7 to 12 carbon atoms, said alkyl, alkenyl and aralkyl radicals being optionally substituted by one or more halogen atoms or by one or more groups $-OR_5$, in which $R_5$ is a hydrogen atom or an alkyl radical having from 1 to 6 carbon atoms, preferably a methyl or ethyl radical; one of $R_1$ or $R_2$ being a hydrogen atom,

or at least a plant extract containing a xanthine of formula (Ia) or a combination of said compound and extract,

in a cosmetically acceptable excipient, vehicle or carrier;

as a cosmetic agent for skin or hair pigmentation or for strengthening the skin's natural defenses against solar radiation.

**21.** Use according to one claim 19 or 20, characterized in that the above mentioned xanthine or the plant extract in which it is present, or the combination of said xanthine and plant extract is at least partially incorporated into hydrated lipidic lamellar phases or in vesicles of liposome type.

**22.** Use according to anyone of claims 19 to 21, characterized in that $R_2$ is a hydrogen atom and $R_1$ is an alkyl radical preferably having from 1 to 4 carbon atoms, notably a methyl, ethyl, propyl, or butyl radical, preferably a methyl radical.

**23.** Use according to anyone of claims 19 to 21, characterized in that $R_1$ is a hydrogen atom and $R_2$ is an alkyl radical with a linear, branched or cyclic chain, preferably having from 1 to 8 carbon atoms, an alkenyl radical with a linear or branched chain preferably having from 2 to 5 carbon atoms, an alkynyl radical having preferably from 2 to 3 carbon atoms, an aralkyl radical having preferably from 7 to 12 carbon atoms, said alkyl, alkenyl and aralkyl radicals being optionally substitued by one or more halogen atoms or by one or more groups $-OR_5$, in which $R_5$ is a hydrogen atom or an alkyl radical having from 1 to 6 carbon atoms, preferably a methyl or ethyl radical.

**24.** Use according to claim 23, characterized in that $R_2$ is an alkyl radical having preferably from 1 to 4 carbon atoms, more preferably a methyl, propyl, butyl or isobutyl radical.

**25.** Use according to anyone of claims 19 to 24, characterized in that the total concentration in compounds of formula (Ia) mentioned above, or their salts, or plant extracts in which they are present, is between 0.001 % and 10 % by weight, preferably between 0.01 % and 1 % by weight, relative to the total weight of the composition.

**26.** Cosmetic or pharmaceutical composition, especially dermatological composition, notably for promoting skin or hair pigmentation, characterized in that it comprises at least one xanthine of formula (Ia) or one of its cosmetic or pharmaceutical salts of formula (Ia):

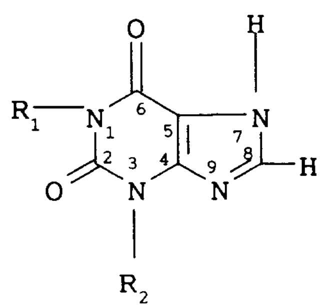

(Ia)

in which:

$R_1$ and $R_2$ are different and are each a hydrogen atom, an alkyl radical with a linear, branched or cyclic chain, preferably having from 1 to 8 carbon atoms, an alkenyl radical with a linear or branched chain, preferably having from 2 to 5 carbon atoms, an alkynyl radical preferably having from 2 to 3 carbon atoms, an aralkyl radical preferably having from 7 to 12 carbon atoms, said alkyl, alkenyl and aralkyl radicals being optionally substituted by one or more halogen atoms or by one or more groups $-OR_5$, in which $R_5$ is a hydrogen atom or an alkyl radical having from 1 to 6 carbon atoms, preferably a methyl or ethyl radical; one among $R_1$ or $R_2$ being a hydrogen atom,

or at least one plant extract containing a xanthine of formula (Ia) or a combination of the above-mentioned compounds and extract, in a cosmetically or pharmaceutically excipient, vehicle or carrier.

**27.** Cosmetic or pharmaceutical composition, especially a dermatological composition, notably for promoting skin and hair pigmentation, characterized in that it comprises an efficient amount for promoting pigmentation of at least a xanthine selected from the group consisting of:

1,8-dimethyl-3-isobutylxanthine,
7-benzyl-1,8-dimethyl-3-isobutylxanthine,
1-(2-hydroxyethyl)-3-isobutyl-8-methylxanthine,
1,8-dimethyl-3-(2-methylprop-2-enyl)xanthine,
3-(2,3-dibromopropyl)-1-ethyl-8-methylxanthine,
1,3-di(prop-2-enyl)-8-methylxanthine,
7-benzyl-8-bromo-3-isobutyl-1-methylxanthine,
8-ethyl-1-(2-hydroxyethyl)-3-isobutylxanthine,
8-hydroxymethyl-3-isobutyl-1-methylxanthine,

3-isobutyl-8-methoxymethyl-1-methylxanthine,
8-(2-carbomethoxyethyl)-3-isobutyl-1-methylxanthine,
3-isobutyl-1-methyl-8-tert-butylxanthine,
3-isobutyl-1-methyl-8-methylaminoxanthine,
1,3-dipropyl-8-(piperidin-1-yl)xanthine,
8-cyclopropyl-1,3-dipropylxanthine,
8-cyclopentyl-1,3-dipropylxanthine,
8-cyclohexyl-1,3-dipropylxanthine,
1,3-dimethyl-8-phenylxanthine,
1,3-dipropyl-8-phenylxanthine,
8-(2-amino-4-chlorophenyl)-1,3-dipropylxanthine,
1,3-diethyl-7-methylxanthine,
1,3-dipropyl-7-methylxanthine,
7-chloromethyl-1,3-dimethylxanthine,
1,3-dimethyl-7-hydroxymethylxanthine,
1,3-dimethyl-7-(morpholin-4-ylmethyl)xanthine,
1,3,7-triethylxanthine,
3,7-diethyl-1-(prop-2-ynyl)xanthine,
7-(2-chloroethyl)-1,3-dimethylxanthine.
3-benzyl-1-methylxanthine,
3-isopentyl-1-methylxanthine,
1-methyl-3-(prop-2-enyl)xanthine,
1-methyl-3-(2-methylprop-2-enyl)xanthine,
3-butyl-1-methylxanthine,
1,3-diethylxanthine,
1-ethyl-3-propylxanthine,
3-butyl-1-ethylxanthine,
1-ethyl-3-(2-methylprop-2-enyl)xanthine,
3-cyclohexyl-1-ethylxanthine,
1-(2-hydroxyethyl)-3-isobutylxanthine,
1,3-dipropylxanthine,
1,3-di-(prop-2-enyl)xanthine,
1,3-dibutylxanthine,
3-ethyl-1-penthylxanthine,
1-benzyl-3-isobutylxanthine,
1-methylxanthine,
1-ethylxanthine,
1-propylxanthine,
1-butylxanthine,
3-methylxanthine,
3-propylxanthine,
3-butylxanthine, and
3-isobutylxanthine,

or a pharmaceutically or cosmetically acceptable salt of this xanthine, at least a plant extract in which it is present, or a combination of the above-mentioned compounds and extracts, in a cosmetically or pharmaceutically acceptable excipient, vehicle or carrier.

**28.** Composition according to one of claims 26 or 27, characterized in that the total concentration of compounds of formula (Ia) mentioned above, or theirs stalts, or the above-mentioned plant extracts in which they are present, is between 0.001% and 10% by weight, preferably between 0.01 and 1% by weight, relative to the total weight of the final composition.

**29.** Composition according to one of claims 26 to 28, characterized in that it is formulated for topical application, in particular as a cream, gel or lotion.

**30.** Composition according to one of claims 26 to 29, characterized in that said xanthine, or one of its cosmetically or pharmaceutically acceptable salts, or at least one plant extract in which it is present, or a combination of the above-mentioned xanthine and extracts, is at least partially incorporated in

hydrated lipidic lamellar phases or in vesicles of the liposome type.

**31.** A method of cosmetic treatment of hair or skin for promoting pigmentation thereof, said method comprising applying to the skin or hair to be pigmented a pigmenting efficient amount of at least one xanthine or formula (I) below:

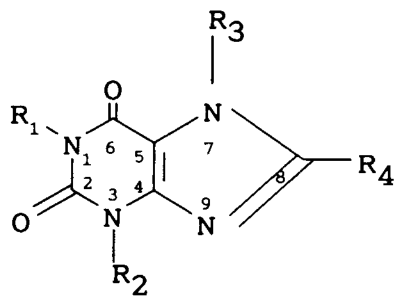

(I)

in which:

$R_1$ and $R_2$ are identical or different and are each a hydrogen atom, an alkyl radical with a linear, branched or cyclic chain, preferably having from 1 to 8 carbon atoms, an alkenyl radical with a linear or branched chain, preferably having from 2 to 5 carbon atoms, an alkynyl radical preferably having from 2 to 3 carbon atoms, an aralkyl radical preferably having from 7 to 12 carbon atoms, said alkyl, alkenyl and aralkyl radicals being optionally substituted by one or more halogen atoms or by one or more groups $-OR_5$, in which $R_5$ is a hydrogen atom or an alkyl radical having from 1 to 6 carbon atoms, preferably a methyl or ethyl radical;

$R_3$ is a hydrogen atom, an alkyl radical preferably having from 1 to 4 carbon atoms, or a benzyl radical, said alkyl radical being optionally substitued by a heterocyclic radical such as the morpholin-4-yl or piperidin-1-yl radical, or by one or more halogen atoms or hydroxyl groups;

$R_4$ is a hydrogen or halogen atom, an alkyl radical with a linear, branched or cyclic chain, preferably having from 1 to 6 carbon atoms, an aryl radical or

a group

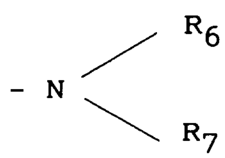

in which $R_6$ and $R_7$ independently are a hydrogen atom, an alkyl radical having from 1 to 6 carbon atoms, preferably a methyl or ethyl radical, or else they form together, with the nitrogen atom, a heterocycle, preferably a saturated monocyclic heterocycle, said alkyl or aryl radicals being optionally substituted by one or more halogen atoms or hydroxyl, methoxy, carbomethoxy or amino groups;

or the use of a cosmetically acceptable salt of this xanthine, or that of a plant extract in which such a xanthine is present.

**32.** A method of cosmetic treatment of skin or hair for promoting pigmentation thereof, said method comprising applying to said skin or hair to be pigmented a pigmenting efficient amount of at least one xanthine of formula (Ia) belows:

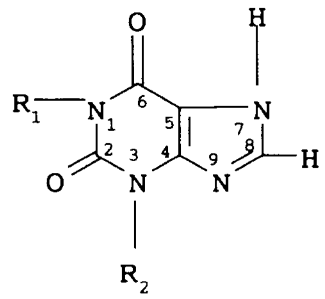

(Ia)

in which:
$R_1$ and $R_2$ are different and are each a hydrogen atom, an alkyl radical with a linear, branched or cyclic chain, preferably having from 1 to 8 carbon atoms, an alkenyl radical with a linear or branched chain, preferably having from 2 to 5 carbon atoms, an alkynyl radical preferably having from 2 to 3 carbon atoms, an aralkyl radical preferably having from 7 to 12 carbon atoms, said alkyl, alkenyl and aralkyl radicals being optionally substituted by one or more halogen atoms or by one or more groups $-OR_5$, in which $R_5$ is a hydrogen atom or an alkyl radical having from 1 to 6 carbon atoms, preferably a methyl or ethyl radical; one among $R_1$ or $R_2$ being a hydrogen atom,
or one of its cosmetically acceptable salts.

**33.** A method of cosmetic treatment of skin or hair for promoting pigmentation thereof, said method comprising applying to said skin or hair to be pigmented a pigmenting efficient amount of at least one xanthine selected from the group consisting of:
1,8-dimethyl-3-isobutylxanthine,
7-benzyl-1,8-dimethyl-3-isobutylxanthine,
1-(2-hydroxyethyl)-3-isobutyl-8-methylxanthine,
1,8-dimethyl-3-(2-methylprop-2-enyl)xanthine,
3-(2,3-dibromopropyl)-1-ethyl-8-methylxanthine,
1,3-di(prop-2-enyl)-8-methylxanthine,
7-benzyl-8-bromo-3-isobutyl-1-methylxanthine,
8-ethyl-1-(2-hydroxyethyl)-3-isobutylxanthine,
8-hydroxymethyl-3-isobutyl-1-methylxanthine,
3-isobutyl-8-methoxymethyl-1-methylxanthine,
8-(2-carbomethoxyethyl)-3-isobutyl-1-methylxanthine,
3-isobutyl-1-methyl-8-tert-butylxanthine,
3-isobutyl-1-methyl-8-methylaminoxanthine,
1,3-dipropyl-8-(piperidin-1-yl)xanthine,
8-cyclopropyl-1,3-dipropylxanthine,
8-cyclopentyl-1,3-dipropylxanthine,
8-cyclohexyl-1,3-dipropylxanthine,
1,3-dimethyl-8-phenylxanthine,
1,3-dipropyl-8-phenylxanthine,
8-(2-amino-4-chlorophenyl)-1,3-dipropylxanthine,
1,3-diethyl-7-methylxanthine,
1,3-dipropyl-7-methylxanthine,
7-chloromethyl-1,3-dimethylxanthine,
1,3-dimethyl-7-hydroxymethylxanthine,
1,3-dimethyl-7-(morpholin-4-ylmethyl)xanthine,
1,3,7-triethylxanthine,
3,7-diethyl-1-(prop-2-ynyl)xanthine,
7-(2-chloroethyl)-1,3-dimethylxanthine.
3-benzyl-1-methylxanthine,
3-isopentyl-1-methylxanthine,
1-methyl-3-(prop-2-enyl)xanthine,
1-methyl-3-(2-methylprop-2-enyl)xanthine,
3-butyl-1-methylxanthine,
1,3-diethylxanthine,

1-ethyl-3-propylxanthine,
3-butyl-1-ethylxanthine,
1-ethyl-3-(2-methylprop-2-enyl)xanthine,
3-cyclohexyl-1-ethylxanthine,
1-(2-hydroxyethyl)-3-isobutylxanthine,
1,3-dipropylxanthine,
1,3-di-(prop-2-enyl)xanthine,
1,3-dibutylxanthine,
3-ethyl-1-penthylxanthine,
1-benzyl-3-isobutylxanthine,
1-methylxanthine,
1-ethylxanthine,
1-propylxanthine,
1-butylxanthine,
3-methylxanthine,
3-propylxanthine,
3-butylxanthine, and
3-isobutylxanthine,
or a cosmetically salt of said xanthine.

**34.** Method according to one of claims 31 to 33, characterized in that said xanthine or one of its cosmetically acceptable salts, or a plant extract containing same, or a combination of said xanthine and of said extract is at least partially incorporated in hydrated lipidic lamellar phases or in vesicles of the liposome type.

**35.** Method according to one of claims 31 to 34, characterized in that said xanthine or one of its cosmetically acceptable salts, or a plant extract containing same or a combination of said xanthine and said extract is applied at a total concentration ranging between 0.001% and 10 % by weight, preferably between 0.01% and 1 % by weight.

## Patentansprüche

**1.** Verwendung von Xanthinen der folgenden Formel (I):

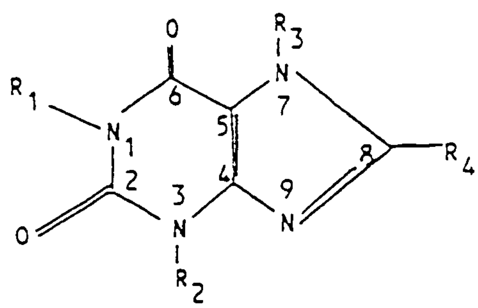

(I),

in der bedeuten:

$R_1$ und $R_2$, die gleich oder verschieden sind, jeweils Wasserstoff, geradkettiges, verzweigtes oder cyclisches Alkyl mit vorzugsweise 1 bis 8 Kohlenstoffatomen, geradkettiges oder verzweigtes Alkenyl mit vorzugsweise 2 bis 5 Kohlenstoffatomen, Alkinyl mit vorzugsweise 2 bis 3 Kohlenstoffatomen oder Aralkyl mit vorzugsweise 7 bis 12 Kohlenstoffatomen, wobei die Alkyl-, Alkenyl- und Aralkylgruppen gegebenenfalls mit einem oder mehreren Halogenatomen oder mit einer oder mehreren Gruppen $-OR_5$ substituiert sind, worin $R_5$ Wasserstoff oder Alkyl mit 1 bis 6 Kohlenstoffatomen, vorzugsweise Methyl oder Ethyl, darstellt,

$R_3$ Wasserstoff, Alkyl mit vorzugsweise 1 bis 4 Kohlenstoffatomen oder Benzyl, wobei die Alkylgruppe gegebenenfalls mit einer heterocyclischen Gruppe, wie 4-Morpholinyl oder 1-Piperidinyl, oder mit einem oder mehreren Halogenatomen oder

einer oder mehreren Hydroxygruppen substituiert ist,
und

$R_4$ Wasserstoff, ein Halogenatom, geradkettiges, verzweigtes oder cyclisches Alkyl mit vorzugsweise 1 bis 6 Kohlenstoffatomen, Aryl oder eine Gruppe

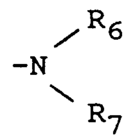

,

worin $R_6$ und $R_7$ unabhängig Wasserstoff, Alkyl mit 1 bis 6 Kohlenstoffatomen, vorzugsweise Methyl oder Ethyl, darstellen oder zusammen und mit dem Stickstoffatom einen vorzugsweise monocyclischen gesättigten Heterocyclus bilden,
wobei die Alkyl- oder Arylgruppen gegebenenfalls mit einem oder mehreren Halogenatomen oder einer oder mehreren Hydroxy-, Methoxy-, Carbomethoxy- oder Aminogruppen substituiert sind,

oder Verwendung von pharmazeutisch oder kosmetisch akzeptablen Salzen dieser Xanthine oder Verwendung von ein solches Xanthin enthaltenden Pflanzenextrakten zur Herstellung von pharmazeutischen und insbesondere dermatologischen Zusammensetzungen zur Förderung der Pigmentierung der Haut oder der Haare oder zur Verstärkung der natürlichen Abwehrkräfte der Haut gegen Sonnenstrahlung.

**2.** Verwendung von Xanthinen der folgenden Formel (I):

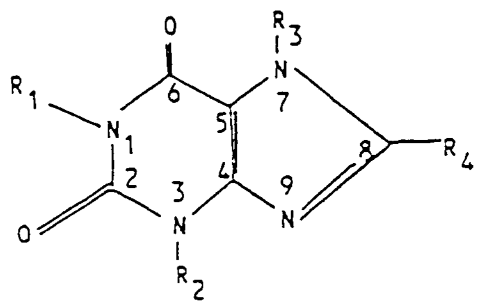

(I),

in der bedeuten:

$R_1$ und R2, die gleich oder verschieden sind, jeweils Wasserstoff, geradkettiges, verzweigtes oder cyclisches Alkyl mit vorzugsweise 1 bis 8 Kohlenstofatomen, Alkenyl mit gerader oder verzweigter Kette mit vorzugsweise 2 bis 5 Kohlenstoffatomen, Alkinyl mit vorzugsweise 2 bis 3 Kohlenstoffatomen oder Aralkyl mit vorzugsweise 7 bis 12 Kohlenstoffatomen,
wobei die Alkyl-, Alkenyl- und Aralkylgruppen gegebenenfalls mit einem oder mehreren Halogenatomen oder mit einer oder mehreren Gruppen -$OR_5$ substituiert sind, worin $R_5$ Wasserstoff oder Alkyl mit 1 bis 6 Kohlenstoffatomen, vorzugsweise Methyl oder Ethyl, darstellt,

$R_3$ Wasserstoff, Alkyl mit vorzugsweise 1 bis 4 Kohlenstoffatomen oder Benzyl,
wobei die Alkylgruppe gegebenenfalls mit einer heterocyclischen Gruppe, wie 4-Morpholinyl oder 1-Piperidinyl, oder mit einem oder mehreren Halogenatomen oder einer oder mehreren Hydroxygruppen substituiert ist,
und

$R_4$ Wasserstoff oder ein Halogenatom, eine geradkettige, verzweigte oder cyclische Alkylgruppe mit vorzugsweise 1 bis 6 Kohlenstoffatomen, Aryl oder eine Gruppe

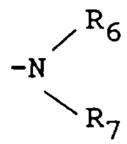 ,

worin $R_6$ und $R_7$ unabhängig Wasserstoff, Alkyl mit 1 bis 6 Kohlenstoffatomen, vorzugsweise Methyl oder Ethyl, darstellen oder zusammen und mit dem Stickstoffatom einen vorzugsweise monocyclischen gesättigten Heterocyclus bilden,

wobei die Alkyl- oder Arylgruppen gegebenenfalls mit einem oder mehreren Halogenatomen oder einer oder mehreren Hydroxy-, Methoxy-, Carbomethoxy- oder Aminogruppen substituiert sind,

oder Verwendung von kosmetisch akzeptablen Salzen dieser Xanthine oder Verwendung von ein solches Xanthin enthaltenden Pflanzenextrakten als kosmetische Mittel zur Pigmentierung der Haut oder der Haare oder zur Verstärkung der natürlichen Abwehrkräfte der Haut gegen Sonnenstrahlung.

**3.** Verwendung nach Anspruch 1 oder 2, dadurch gekennzeichnet, daß das oben angegebene Xanthin der Formel (I) oder eines seiner kosmetisch oder pharmazeutisch akzeptablen Salze oder der sie enthaltende Pflanzenextrakt oder die Kombination des Xanthins mit dem oben angeführten Pflanzenextrakt mindestens zum Teil in wasserhaltigen lamellaren Lipidphasen oder Vesikeln vom Liposomentyp eingebracht werden.

**4.** Verwendung nach einem der Ansprüche 1 bis 3, dadurch gekennzeichnet, daß das verwendete Xanthin ein Xanthin der oben angegebenen Formel (I) ist, in der bedeuten:

$R_1$ Wasserstoff oder Methyl, Ethyl, Propyl, Butyl, Isobutyl, Pentyl, Isopentyl, 2-Propenyl, 2-Propinyl, Benzyl, Hydroxymethyl, 2-Hydroxyethyl, 2-Hydroxypropyl oder 3-Hydroxypropyl,

$R_2$ Wasserstoff oder Methyl, Ethyl, Propyl, Isopropyl, Butyl, Isobutyl, 2-Propenyl, 2-Methyl-2-propenyl, Cyclohexyl, Benzyl oder 2,3-Dibrompropyl,

$R_3$ Wasserstoff oder Methyl, Chlormethyl, Hydroxymethyl, 2-Chlorethyl, 4-Morpholinylmethyl oder Benzyl und

$R_4$ Wasserstoff oder Brom oder Methyl, Ethyl, Cyclopropyl, Cyclopentyl, Cyclohexyl, tert.-Butyl, 2-Carbomethoxyethyl, Hydroxymethyl, Methoxymethyl, Methylamino oder 1-Piperidinyl.

**5.** Verwendung nach einem der Ansprüche 1 bis 4, dadurch gekennzeichnet, daß das Xanthin der Formel (I) ausgewählt wird unter:

Xanthin,
1,8-Dimethyl-3-isobutylxanthin,
7-Benzyl-1,8-dimethyl-3-isobutylxanthin,
1-(2-Hydroxyethyl)-3-isobutyl-8-methylxanthin,
1,8-Dimethyl-3-(2-methyl-2-propenyl)-xanthin,
3-(2,3-Dibrompropyl)-1-ethyl-8-methylxanthin,
1,3-Di(2-propenyl)-8-methylxanthin,
7-Benzyl-8-brom-3-isobutyl-1-methylxanthin,
8-Ethyl-1-(2-hydroxyethyl)-3-isobutylxanthin,
8-Hydroxymethyl-3-isobutyl-1-methylxanthin,
3-Isobutyl-8-methoxymethyl-1-methylxanthin,
8-(2-Carbomethoxyethyl)-3-isobutyl-1-methylxanthin,
3-Isobutyl-1-methyl-8-tert.-butylxanthin,
3-Isobutyl-1-methyl-8-methylaminoxanthin,
1,3-Dipropyl-8-(1-piperidinyl)-xanthin,
8-Cyclopropyl-1,3-dipropylxanthin,
8-Cyclopentyl-1,3-dipropylxanthin,
8-Cyclohexyl-1,3-dipropylxanthin,
1,3-Dimethyl-8-phenylxanthin,
1,3-Dipropyl-8-phenylxanthin,
8-(2-Amino-4-chlorphenyl)-1,3-dipropylxanthin,
1,3-Diethyl-7-methylxanthin,
1,3-Dipropyl-7-methylxanthin,

7-Chlormethyl-1,3-dimethylxanthin,
1,3-Dimethyl-7-hydroxymethylxanthin,
1,3-Dimethyl-7-(morpholin-4-ylmethyl)-xanthin,
1,3,7-Triethylxanthin,
3,7-Diethyl-1-(2-propinyl)-xanthin,
7-(2-Chlorethyl)-1,3-dimethylxanthin.

**6.** Verwendung nach Anspruch 5, dadurch gekennzeichnet, daß das xanthin ausgewählt wird unter:
1,8-Dimethyl-3-isobutylxanthin,
8-(2-Carbomethoxyethyl)-3-isobutylxanthin und
3-Isobutyl-1-methyl-8-tert.-butylxanthin.

**7.** Verwendung nach einem der Ansprüche 1 bis 4, dadurch gekennzeichnet, daß in der oben angegebenen Formel (I) $R_3$ und $R_4$ jeweils Wasserstoff und $R_1$ und $R_2$ jeweils, unabhängig voneinander, Wasserstoff oder eine wie zuvor definierte Gruppe darstellen.

**8.** Verwendung nach Anspruch 7, dadurch gekennzeichnet, daß das oben angegebene Xanthin ausgewählt wird unter:
3-Isobutyl-1-methylxanthin,
3-Benzyl-1-methylxanthin,
3-Isopentyl-1-methylxanthin,
1-Methyl-3-(2-propenyl)-xanthin,
1-Methyl-3-(2-methyl-2-propenyl)-xanthin,
3-Butyl-1-methylxanthin,
1,3-Diethylxanthin,
1-Ethyl-3-propylxanthin,
3-Butyl-1-ethylxanthin,
1-Ethyl-3-(2-methyl-2-propenyl)-xanthin,
3-Cyclohexyl-1-ethylxanthin,
1-(2-Hydroxyethyl)-3-isobutylxanthin,
1,3-Dipropylxanthin,
1,3-Di(2-propenyl)-xanthin,
1,3-Dibutylxanthin,
3-Ethyl-1-pentylxanthin und
1-Benzyl-3-isobutylxanthin.

**9.** Verwendung nach Anspruch 8, dadurch gekennzeichnet, daß das Xanthin ausgewählt wird unter:
3-Isobutyl-1-methylxanthin,
3-Benzyl-1-methylxanthin,
1,3-Diethylxanthin,
1-Ethyl-3-propylxanthin,
3-Butyl-1-ethylxanthin,
1,3-Dipropylxanthin und
1,3-Dibutylxanthin.

**10.** Verwendung nach einem der Ansprüche 1 bis 3, dadurch gekennzeichnet, daß 1,3-Dimethylxanthin oder 3,7-Dimethylxanthin verwendet werden.

**11.** Verwendung nach einem der Ansprüche 1 bis 3, dadurch gekennzeichnet, daß 3-Isobutyl-1-methylxanthin verwendet wird.

**12.** Verwendung nach einem der Ansprüche 1 bis 3, dadurch gekennzeichnet, daß in Formel (I) $R_3$ und $R_4$ jeweils Wasserstoff darstellen und $R_1$ und $R_2$ Wasserstoff oder eine wie zuvor definierte Gruppe darstellen, wobei zumindest $R_1$ oder $R_2$ Wasserstoff darstellen.

**13.** Verwendung nach Anspruch 12, dadurch gekennzeichnet, daß $R_2$, $R_3$ und $R_4$ jeweils Wasserstoff und $R_1$ Alkyl mit vorzugsweise 1 bis 4 Kohlenstoffatomen, insbesondere Methyl, Ethyl, Propyl oder Butyl und vorzugsweise Methyl, darstellen.

**14.** Verwendung nach Anspruch 12, dadurch gekennzeichnet, daß in Formel (I) $R_1$, $R_3$ und $R_4$ jeweils Wasserstoff und $R_2$ eine wie zuvor definierte Gruppe, insbesondere Alkyl mit vorzugsweise 1 bis 4 Kohlenstoffatomen, darstellen.

**15.** Verwendung nach Anspruch 14, dadurch gekennzeichnet, daß $R_2$ Methyl, Propyl, Butyl oder Isobutyl darstellt.

**16.** Verwendung nach einem der Ansprüche 1 bis 15, dadurch gekennzeichnet, daß das pharmazeutisch oder kosmetisch akzeptable Salz ein Alkalisalz, wie ein Natrium-, Kalium- oder Ammoniumsalz, oder ein Salz einer organischen Base wie Ethanolamin, Diethanolamin, Ethylendiamin, Isopropylamin, Triethanolamin, Octadecylamin oder Cholin, oder ein Salz einer Aminosäure, wie Glycin oder Lysin, ist.

**17.** Verwendung nach einem der Ansprüche 1 bis 16, dadurch gekennzeichnet, daß mindestens ein Xanthin der Formel (I) oder eines seiner wie zuvor definierten Salze in Kombination mit mindestens einem vorgenannten Pflanzenextrakt verwendet wird.

**18.** Verwendung nach einem der Ansprüche 1 bis 17, dadurch gekennzeichnet, daß die Gesamtkonzentration an Verbindungen der Formel (I) oder ihren Salzen oder an diese enthaltenden Pflanzenextrakten 0,001 bis 10 Gew.-% und vorzugsweise 0,01 bis 1 Gew.-%, bezogen auf das Gesamtgewicht der Zusammensetzung, beträgt.

**19.** Verwendung von Xanthinen der Formel (Ia):

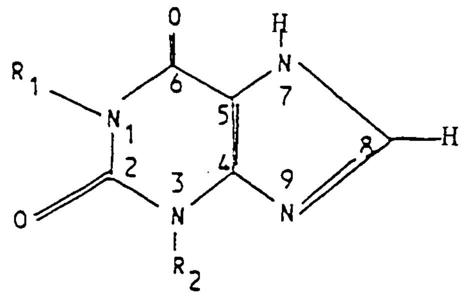

(Ia),

in der bedeuten:

$R_1$ und $R_2$, die verschieden sind, jeweils Wasserstoff, geradkettiges, verzweigtes oder cyclisches Alkyl mit vorzugsweise 1 bis 8 Kohlenstoffatomen, geradkettiges oder verzweigtes Alkenyl mit vorzugsweise 2 bis 5 Kohlenstoffatomen, Alkinyl mit vorzugsweise 2 bis 3 Kohlenstoffatomen oder Aralkyl mit vorzugsweise 7 bis 12 Kohlenstoffatomen, wobei die Alkyl-, Alkenyl- und Aralkylgruppen gegebenenfalls mit einem oder mehreren Halogenatomen oder mit einer oder mehreren Gruppen $-OR_5$ substituiert sind, worin $R_5$ Wasserstoff oder Alkyl mit 1 bis 6 Kohlenstoffatomen, vorzugsweise Methyl oder Ethyl, darstellt und einer der Substituenten $R_1$ und $R_2$ Wasserstoff ist,

oder von pharmazeutisch akzeptablen Salze davon oder mindestens eines Pflanzenextrakts, der ein Xanthin der Formel (Ia) enthält, oder einer Kombination dieser Verbindungen mit Pflanzenextrakten wie oben angegeben in einem pharmazeutisch akzeptablen Excipiens, Vehikel oder Träger

zur Herstellung von pharmazeutischen und insbesondere dermatologischen Zusammensetzungen zur Förderung der Pigmentierung der Haut oder der Haare oder zur Verstärkung der natürlichen Abwehrkräfte der Haut gegen Sonnenstrahlung.

**20.** Verwendung von Xanthinen der Formel (Ia):

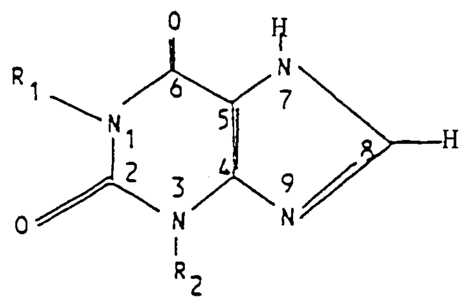

(Ia),

in der bedeuten:

$R_1$ und $R_2$, die verschieden sind, jeweils Wasserstoff, geradkettiges, verzweigtes oder cyclisches Alkyl mit vorzugsweise 1 bis 8 Kohlenstoffatomen, geradkettiges oder verzweigtes Alkenyl mit vorzugsweise 2 bis 5 Kohlenstoffatomen, Alkinyl mit vorzugsweise 2 bis 3 Kohlenstoffatomen oder Aralkyl mit vorzugsweise 7 bis 12 Kohlenstoffatomen, wobei die Alkyl-, Alkenyl- und Aralkylgruppen gegebenenfalls mit einem oder mehreren Halogenatomen oder mit einer oder mehreren Gruppen $-OR_5$ substituiert sind, worin $R_5$ Wasserstoff oder Alkyl mit 1 bis 6 Kohlenstoffatomen, vorzugsweise Methyl oder Ethyl, darstellt und einer der Substituenten $R_1$ und $R_2$ Wasserstoff ist,

oder von kosmetisch akzeptablen Salze davon oder mindestens eines Pflanzenextrakts, der ein Xanthin der Formel (Ia) enthält, oder einer Kombination dieser Verbindungen mit Pflanzenextrakten wie oben angegeben in einem kosmetisch akzeptablen Excipiens, Vehikel oder Träger
als kosmetisches Mittel zur Pigmentierung der Haut oder der Haare oder zur Verstärkung der natürlichen Abwehrkräfte der Haut gegen Sonnenstrahlung.

**21.** Verwendung nach Anspruch 19 oder 20, dadurch gekennzeichnet, daß das vorgenannte Xanthin oder der das Xanthin enthaltende Pflanzenextrakt oder die Kombination des Xanthins mit dem vorgenannten Pflanzenextrakt zumindest teilweise in wasserhaltigen lamellaren Lipidphasen oder in Vesikeln vom Liposomentyp eingebracht werden.

**22.** Verwendung nach einem der Ansprüche 19 bis 21, dadurch gekennzeichnet, daß $R_2$ Wasserstoff und $R_1$ Alkyl mit vorzugsweise 1 bis 4 Kohlenstoffatomen, insbesondere Methyl, Ethyl, Propyl oder Butyl und vorzugsweise Methyl, darstellen.

**23.** Verwendung nach einem der Ansprüche 19 bis 21, dadurch gekennzeichnet, daß $R_1$ Wasserstoff und $R_2$ geradkettiges verzweigtes oder cyclisches Alkyl mit vorzugsweise 1 bis 8 Kohlenstoffatomen, geradkettiges oder verzweigtes Alkenyl mit vorzugsweise 2 bis 5 Kohlenstoffatomen, Alkinyl mit vorzugsweise 2 bis 3 Kohlenstoffatomen oder Aralkyl mit vorzugsweise 7 bis 12 Kohlenstoffatomen bedeuten, wobei die Alkyl-, Alkenyl und Aralkylgruppen gegebenenfalls mit einem oder mehreren Halogenatomen oder mit einer oder mehreren Gruppen $-OR_5$ substituiert sind, worin $R_5$ Wasserstoff oder Alkyl mit 1 bis 6 Kohlenstoffatomen, vorzugsweise Methyl oder Ethyl, darstellt.

**24.** Verwendung nach Anspruch 23, dadurch gekennzeichnet, daß $R_2$ Alkyl mit vorzugsweise 1 bis 4 Kohlenstoffatomen und noch bevorzugter Methyl, Propyl, Butyl oder Isobutyl darstellt.

**25.** Verwendung nach einem der Ansprüche 19 bis 24, dadurch gekennzeichnet, daß die Gesamtkonzentration an Verbindungen der oben angegebenen Formel (Ia) oder ihren Salzen oder an diese enthaltenden Pflanzenextrakten 0,001 bis 10 Gew.-% und vorzugsweise 0,1 bis 1 Gew.-%, bezogen auf das Gesamtgewicht der Zusammensetzung, beträgt.

**26.** Kosmetische oder pharmazeutische und insbesondere dermatologische Zusammensetzung zur Begünstigung der Pigmentierung der Haut oder der Haare, dadurch gekennzeichnet, daß sie mindestens ein Xanthin der Formel (Ia)

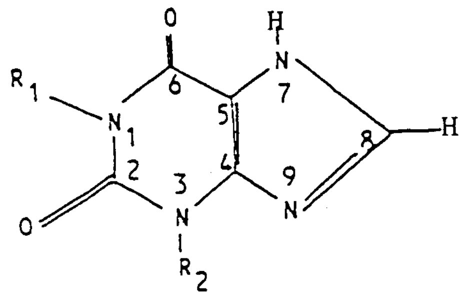

(Ia),

in der bedeuten:

$R_1$ und $R_2$, die verschieden sind, jeweils Wasserstoff, geradkettiges, verzweigtes oder cyclisches Alkyl mit vorzugsweise 1 bis 8 Kohlenstoffatomen, geradkettiges oder verzweigtes Alkenyl mit vorzugsweise 2 bis 5 Kohlenstoffatomen, Alkinyl mit vorzugsweise 2 bis 3 Kohlenstoffatomen oder Aralkyl mit vorzugsweise 7 bis 12 Kohlenstoffatomen, wobei die Alkyl-, Alkenyl- und Aralkylgruppen gegebenenfalls mit einem oder mehreren Halogenatomen oder mit einer oder mehreren Gruppen $-OR_5$ substituiert sind, worin $R_5$ Wasserstoff oder Alkyl mit 1 bis 6 Kohlenstoffatomen, vorzugsweise Methyl oder Ethyl, darstellt und einer der Substituenten Wasserstoff ist,

oder mindestens ein kosmetisch oder pharmazeutisch akzeptables Salz davon oder mindestens einen Pflanzenextrakt, der ein Xanthin der Formel (Ia) enthält, oder eine Kombination dieser Verbindungen mit Pflanzenextrakten wie oben angegeben, in einem kosmetisch oder pharmazeutisch akzeptablen Excipiens, Vehikel oder Träger enthält.

**27.** Kosmetische oder pharmazeutische und insbesondere dermatologische Zusammensetzung, besonders zur Förderung der Pigmentierung der Haut oder der Haare,
dadurch gekennzeichnet, daß sie eine zur Erzeugung der Pigmentierung wirksame Menge mindestens eines Xanthins, das ausgewählt ist unter:
1,8-Dimethyl-3-isobutylxanthin,
7-Benzyl-1,8-dimethyl-3-isobutylxanthin,
1-(2-Hydroxyethyl)-3-isobutyl-8-methylxanthin,
1,8-Dimethyl-3-(2-methyl-2-propenyl)-xanthin,
3-(2,3-Dibrompropyl)-1-ethyl-8-methylxanthin,
1,3-Di(2-propenyl)-8-methylxanthin,
7-Benzyl-8-brom-3-isobutyl-1-methylxanthin,
8-Ethyl-1-(2-hydroxyethyl)-3-isobutylxanthin,
8-Hydroxymethyl-3-isobutyl-1-methylxanthin,
3-Isobutyl-8-methoxymethyl-1-methylxanthin,
8-(2-Carbomethoxyethyl)-3-isobutyl-1-methylxanthin,
3-Isobutyl-1-methyl-8-tert.-butylxanthin,
3-Isobutyl-1-methyl-8-methylaminoxanthin,
1,3-Dipropyl-8-(1-piperidinyl)-xanthin,
8-Cyclopropyl-1,3-dipropylxanthin,
8-Cyclopentyl-1,3-dipropylxanthin,
8-Cyclohexyl-1,3-dipropylxanthin,
1,3-Dimethyl-8-phenylxanthin,
1,3-Dipropyl-8-phenylxanthin,
8-(2-Amino-4-chlorphenyl)-1,3-dipropylxanthin,
1,3-Diethyl-7-methylxanthin,
1,3-Dipropyl-7-methylxanthin,
7-Chlormethyl-1,3-dimethylxanthin,
1,3-Dimethyl-7-hydroxymethylxanthin,
1,3-Dimethyl-7-(morpholin-4-ylmethyl)-xanthin,
1,3,7-Triethylxanthin,
3,7-Diethyl-1-(2-propinyl)-xanthin,
7-(2-Chlorethyl)-1,3-dimethylxanthin,

3-Benzyl-1-methylxanthin,
3-Isopentyl-1-methylxanthin,
1-Methyl-3-(2-propenyl)-xanthin,
1-Methyl-3-(2-methyl-2-propenyl)-xanthin,
3-Butyl-1-methylxanthin,
1,3-Diethylxanthin,
1-Ethyl-3-propylxanthin,
3-Butyl-1-ethylxanthin,
1-Ethyl-3-(2-methyl-2-propenyl)-xanthin,
3-Cyclohexyl-1-ethylxanthin,
1-(2-Hydroxyethyl)-3-isobutylxanthin,
1,3-Dipropylxanthin,
1,3-Di(2-propenyl)-xanthin,
1,3-Dibutylxanthin,
3-Ethyl-1-pentylxanthin,
1-Benzyl-3-isobutylxanthin,
1-Methylxanthin,
1-Ethylxanthin,
1-Propylxanthin,
1-Butylxanthin,
3-Methylxanthin,
3-Propylxanthin,
3-Butylxanthin und
3-Isobutylxanthin,
oder eines pharmazeutisch oder kosmetisch akzeptablen Salzes dieses Xanthins oder mindestens einen ein solches Xanthin enthaltenden Pflanzenextrakt oder eine Kombination der oben angegebenen Verbindungen und Pflanzenextrakte
in einem kosmetisch oder pharmazeutisch akzeptablen Excipiens, Vehikel oder Träger enthält.

**28.** Zusammensetzung nach Anspruch 26 oder 27, dadurch gekennzeichnet, daß die Gesamtkonzentration an Verbindungen der oben angegebenen Formel (Ia) oder ihren Salzen oder an diese Verbindungen enthaltenden, vorgenannten Pflanzenextrakten 0,001 bis 10 Gew.-% und vorzugsweise 0,01 bis 1 Gew.-%, bezogen auf das Gesamtgewicht der fertigen Zusammensetzung, beträgt.

**29.** Zusammensetzung nach einem der Ansprüche 26 bis 28, dadurch gekennzeichnet, daß sie zur topischen Anwendung, insbesondere als Creme, Gel oder Lotion, formuliert ist.

**30.** Zusammensetzung nach einem der Ansprüche 26 bis 29, dadurch gekennzeichnet, daß das vorgenannte Xanthin oder eines seiner kosmetisch oder pharmazeutisch akzeptablen Salze oder mindestens ein sie enthaltender Pflanzenextrakt oder eine Kombination des Xanthins mit dem Pflanzenextrakt wie oben angegeben mindestens zum Teil in wasserhaltigen lamellaren Lipidphasen oder Vesikeln vom Liposomentyp eingebracht ist.

**31.** Verfahren zur kosmetischen Behandlung der Haut oder der Haare zur Förderung der Pigmentierung, das die Anwendung einer zur Erzeugung der Pigmentierung wirksamen Menge mindestens eines Xanthins der folgenden Formel (I):

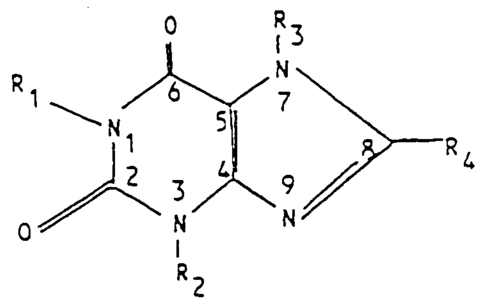

(I),

in der bedeuten:

$R_1$ und $R_2$, die gleich oder verschieden sind, jeweils Wasserstoff, geradkettiges, verzweigtes oder cyclisches Alkyl mit vorzugsweise 1 bis 8 Kohlenstoffatomen, geradkettiges oder verzweigtes Alkenyl mit vorzugsweise 2 bis 5 Kohlenstoffatomen, Alkinyl mit vorzugsweise 2 bis 3 Kohlenstoffatomen oder Aralkyl mit vorzugsweise 7 bis 12 Kohlenstoffatomen, wobei die Alkyl-, Alkenyl- und Aralkylgruppen gegebenenfalls mit einem oder mehreren Halogenatomen oder mit einer oder mehreren Gruppen $-OR_5$ substituiert sind, worin $R_5$ Wasserstoff oder Alkyl mit 1 bis 6 Kohlenstoffatomen, vorzugsweise Methyl oder Ethyl, darstellt,

$R_3$ Wasserstoff, Alkyl mit vorzugsweise 1 bis 4 Kohlenstoffatomen oder Benzyl, wobei die Alkylgruppe gegebenenfalls mit einer heterocyclischen Gruppe, wie 4-Morpholinyl oder 1-Piperidinyl, oder mit einem oder mehreren Halogenatomen oder einer oder mehreren Hydroxygruppen substituiert ist, und

$R_4$ Wasserstoff, ein Halogenatom, geradkettiges, verzweigtes oder cyclisches Alkyl mit vorzugsweise 1 bis 6 Kohlenstoffatomen, Aryl oder eine Gruppe

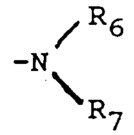

,

worin $R_6$ und $R_7$ unabhängig Wasserstoff, Alkyl mit 1 bis 6 Kohlenstoffatomen, vorzugsweise Methyl oder Ethyl, darstellen oder zusammen und mit dem Stickstoffatom einen vorzugsweise monocyclischen gesättigten Heterocyclus bilden, wobei die Alkyl- oder Arylgruppen gegebenenfalls mit einem oder mehreren Halogenatomen oder einer oder mehreren Hydroxy-, Methoxy-, Carbomethoxy- oder Aminogruppen substituiert sind,

oder eines kosmetisch akzeptablen Salzes dieser Xanthine oder eines ein solches Xanthin enthaltenden Pflanzenextrakts auf die zu pigmentierende Haut oder die zu pigmentierenden Haare umfaßt.

**32.** Verfahren zur kosmetischen Behandlung der Haut oder der Haare zur Förderung der Pigmentierung, das die Anwendung einer zur Erzeugung der Pigmentierung wirksamen Menge mindestens eines Xanthins der folgenden Formel (Ia)

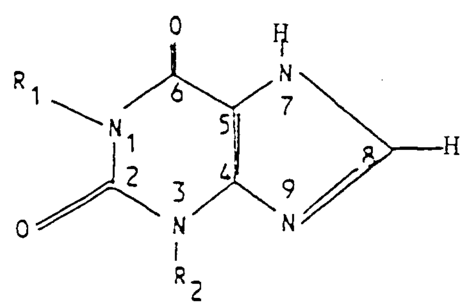

(Ia),

in der bedeuten:

$R_1$ und $R_2$, die verschieden sind, jeweils Wasserstoff, geradkettiges, verzweigtes oder cyclisches Alkyl mit vorzugsweise 1 bis 8 Kohlenstoffatomen, geradkettiges oder verzweigtes Alkenyl mit vorzugsweise 2 bis 5 Kohlenstoffatomen, Alkinyl mit vorzugsweise 2 bis 3 Kohlenstoffatomen oder Aralkyl mit vorzugsweise 7 bis 12 Kohlenstoffatomen, wobei die Alkyl-, Alkenyl- und Aralkylgruppen gegebenenfalls mit einem oder mehreren Halogenatomen oder mit einer oder mehreren Gruppen $-OR_5$ substituiert sind, worin $R_5$ Wasserstoff oder Alkyl mit 1 bis 6 Kohlenstoffatomen, vorzugsweise Methyl oder Ethyl, darstellt, und einer der Substituenten $R_1$ und $R_2$ Wasserstoff ist,

oder von einem ihrer pharmazeutisch akzeptablen Salze auf die zu pigmentierende Haut oder die zu pigmentierenden Haare umfaßt.

**33.** Verfahren zur kosmetischen Behandlung der Haut oder der Haare zur Förderung der Pigmentierung, das die Anwendung einer zur Erzeugung der Pigmentierung wirksamen Menge mindestens eines Xanthins auf die zu pigmentierende Haut oder die zu pigmentierenden Haare umfaßt, das ausgewählt ist unter:
1,8-Dimethyl-3-isobutylxanthin,
7-Benzyl-1,8-dimethyl-3-isobutylxanthin,
1-(2-Hydroxyethyl)-3-isobutyl-8-methylxanthin,
1,8-Dimethyl-3-(2-methyl-2-propenyl)-xanthin,
3-(2,3-Dibrompropyl)-1-ethyl-8-methylxanthin,
1,3-Di(2-propenyl)-8-methylxanthin,
7-Benzyl-8-brom-3-isobutyl-1-methylxanthin,
8-Ethyl-1-(2-hydroxyethyl)-3-isobutylxanthin,
8-Hydroxymethyl-3-isobutyl-1-methylxanthin,
3-Isobutyl-8-methoxymethyl-1-methylxanthin,
8-(2-Carbomethoxyethyl)-3-isobutyl-1-methylxanthin,
3-Isobutyl-1-methyl-8-tert.-butylxanthin,
3-Isobutyl-1-methyl-8-methylaminoxanthin,
1,3-Dipropyl-8-(1-piperidinyl)-xanthin,
8-Cyclopropyl-1,3-dipropylxanthin,
8-Cyclopentyl-1,3-dipropylxanthin,
8-Cyclohexyl-1,3-dipropylxanthin,
1,3-Dimethyl-8-phenylxanthin,
1,3-Dipropyl-8-phenylxanthin,
8-(2-Amino-4-chlorphenyl)-1,3-dipropylxanthin,
1,3-Diethyl-7-methylxanthin,
1,3-Dipropyl-7-methylxanthin,
7-Chlormethyl-1,3-dimethylxanthin,
1,3-Dimethyl-7-hydroxymethylxanthin,
1,3-Dimethyl-7-(morpholin-4-ylmethyl)-xanthin,
1,3,7-Triethylxanthin,
3,7-Diethyl-1-(2-propinyl)-xanthin,
7-(2-Chlorethyl)-1,3-dimethylxanthin,
3-Benzyl-1-methylxanthin,
3-Isopentyl-1-methylxanthin,
1-Methyl-3-(2-propenyl)-xanthin,
1-Methyl-3-(2-methyl-2-propenyl)-xanthin,
3-Butyl-1-methylxanthin,
1,3-Diethylxanthin,
1-Ethyl-3-propylxanthin,
3-Butyl-1-ethylxanthin,
1-Ethyl-3-(2-methyl-2-propenyl)-xanthin,
3-Cyclohexyl-1-ethylxanthin,
1-(2-Hydroxyethyl)-3-isobutylxanthin,
1,3-Dipropylxanthin,
1,3-Di(2-propenyl)-xanthin,
1,3-Dibutylxanthin,
3-Ethyl-1-pentylxanthin,
1-Benzyl-3-isobutylxanthin,
1-Methylxanthin,
1-Ethylxanthin,
1-Propylxanthin,
1-Butylxanthin,
3-Methylxanthin,
3-Propylxanthin,
3-Butylxanthin und

3-Isobutylxanthin
sowie den kosmetisch akzeptablen Salzen dieser Xanthine.

**34.** Verfahren nach einem der Ansprüche 31 bis 33, dadurch gekennzeichnet, daß das oben angegebene Xanthin der Formel (I) oder eines seiner kosmetisch akzeptablen Salze oder ein sie enthaltender Pflanzenextrakt oder eine Kombination dieses Xanthins mit dem oben angeführten Pflanzenextrakt mindestens zum Teil in wasserhaltigen lamellaren Lipidphasen oder Vesikeln vom Liposomentyp eingebracht werden.

**35.** Verfahren nach einem der Ansprüche 31 bis 34, dadurch gekennzeichnet, daß das vorgenannte Xanthin oder eines seiner kosmetisch akzeptablen Salze oder ein das Xanthin enthaltender Pflanzenextrakt oder eine Kombination des Xanthins mit dem vorgenannten Pflanzenextrakt in einer Gesamtkonzentration angewandt wird, die 0,001 bis 10 Gew.-% und vorzugsweise 0,01 bis 1 Gew.-% beträgt.